# EUROPÄISCHE PATENTSCHRIFT

(11) **EP 2 249 804 B1**
(45) Veröffentlichungstag und Bekanntmachung des Hinweises auf die Patenterteilung: **14.12.2016**
(21) Anmeldenummer: 09711314.6
(22) Anmeldetag: 11.02.2009
(51) Int. Cl.: A61K 9/00, A61K 9/06, A61K 9/50, A61K 9/51, A61K 9/70, A61F 13/00, A61K 47/48, A61L 31/00, A61K 31/00

(54) **IMPLANTIERBARE NANOPARTIKEL-ENTHALTENDE PRODUKTE**
IMPLANTABLE PRODUCTS COMPRISING NANOPARTICLES
PRODUITS IMPLANTABLES CONTENANT DES NANOPARTICULES

(30) Priorität: 11.02.2008 DE 102008008522; 11.04.2008 US 71084 P
(43) Veröffentlichungstag der Anmeldung: 17.11.2010
(73) Patentinhaber: MagForce AG, 12489 Berlin (DE)
(72) Erfinder: THIESEN, Burghard, 10555 Berlin (DE); JORDAN, Andreas, 14513 Teltow (DE)
(74) Vertreter: Bösl, Raphael Konrad
(86) Internationale Anmeldenummer: PCT/DE2009/000196
(87) Internationale Veröffentlichungsnummer: WO 2009/100716

(56) Entgegenhaltungen:
- WO-A1-01/05586
- WO-A1-2006/125452
- WO-A2-2004/045652
- WO-A2-2006/015317
- WO-A2-2006/037113
- DE-A1-102005 016 873

## Beschreibung

Die vorliegende Erfindung betrifft Nanopartikel-enthaltende implantierbare Produkte und deren Verwendung in der Medizin insbesondere zur thermotherapeutischen Nachbehandlung nach chirurgischer Entfernung von Tumoren und Krebsgeschwüren.

Nach einer operativen Entfernung von Tumorgewebe ergibt sich fast immer das Problem, dass noch Tumorzellen im Körper verbleiben (unvollständige Resektion). Nach Verschließen der Wunde können diese Tumorzellen wieder zu einem größeren Tumor heranwachsen und/oder metastasieren. Aus diesem Grund erfolgen die für den Patienten sehr belastenden chemotherapeutischen Nachbehandlungen. Da jedoch so wenig wie möglich gesundes Gewebe entfernt werden soll, muss der Operateur einen Kompromiss zwischen einer möglichst vollständigen Tumorentfernung und der geringstmöglichen Entnahme von gesundem Gewebe erzielen.

Aufgabe der vorliegenden Erfindung ist es, Produkte und Verfahren für eine effektivere Nachbehandlung nach Krebsoperationen bereitzustellen.

Die Aufgabe wird durch die unabhängigen Patentansprüche gelöst. Weitere vorteilhafte Ausgestaltungen ergeben sich aus den abhängigen Ansprüchen, den Beispielen und der Beschreibung.

Überraschend wurde gefunden, dass implantierbare und im magnetischen Wechselfeld erwärmbare Nanopartikel enthaltende Medizinprodukte eine im Vergleich zur Chemotherapie deutlich verbesserte Nachbehandlung bei Krebsoperationen ermöglichen, wenn diese Medizinprodukte in den Operationsbereich implantiert oder eingebracht werden.

Die vorliegende Erfindung betrifft somit ein durch ein magnetisches Wechselfeld erwärmbares, festes oder gelartiges Medizinprodukt zur Verwendung in der Nachbehandlung des operierten Bereichs bei Krebsoperationen, wobei das Medizinprodukt in Form eines physiologisch verträglichen Gewebes, Schwammes oder Films vorliegt und wobei magnetische Partikel in dem Medizinprodukt enthalten sind, welche angeregt durch ein magnetisches Wechselfeld Wärme erzeugen und dadurch das Medizinprodukt erwärmen.

Entscheidend bei dem erfindungsgemäßen Medizinprodukt ist, dass die Partikel, d.h. die im magnetischen Wechselfeld anregbaren Partikel, ortsfest in dem Medizinprodukt eingelagert oder angehaftet sind.

Herkömmlich werden wässrige Lösungen von magnetischen Partikeln hergestellt, um entweder die Partikel beladen mit pharmakologischen Wirkstoffen durch ein statischen Magnetfeld an einen bestimmten Wirkort zu dirigieren oder es werden wässrige Lösungen von im magnetischen Wechselfeld anregbaren Partikeln direkt in einen Tumor injiziert, damit sich die Partikel in den Tumorzellen anreichern und durch Wärmeerzeugung die Tumorzellen zerstören. Die Wärme entsteht vorrangig durch Hysteresewärmeverluste der Partikel.

Bei den erfindungsgemäßen Medizinprodukten handelt es sich nicht um wässrige oder physiologische wässrige Lösungen oder Suspensionen der Magnetpartikel, sondern um feste oder gelartige Träger wie z.B. ein Gewebe oder ein Film, worin die Partikel fest eingelagert sind. Sofern es sich um nicht biologisch abbaubare Medizinprodukte handelt, verbleiben die Partikel dauerhaft in dem Medizinprodukt und nach Implantation verbleibt das Medizinprodukt dauerhaft an der implantierten Position, ähnlich eines Dentalimplantats oder künstlichen Kniegelenks.

Weil die Partikel dauerhaft in dem Medizinprodukt verbleiben, nicht durch Diffusion freigesetzt werden und nur im Falle von biologisch abbaubaren Medizinprodukten durch den Abbauprozess freigesetzt werden, kann der Bereich, wo sich das implantierte Medizinprodukt befindet auch noch nach beliebigen Zeiträumen, d.h. nach einer Woche nach Implantation, nach einem Monat nach Implantation, nach einem Jahr nach Implantation, als auch noch nach 10 Jahren nach Implantation erwärmt werden.

Bevorzugte Ausführungsformen der vorliegenden Erfindung betreffen biologisch abbaubare Medizinprodukte, welche sich je nach Indikation unterschiedlich schnell im menschlichen und tierischen Körper abbauen lassen. Aus diesen Medizinprodukten werden die Partikel aber auch nicht durch Diffusion sondern nur im Rahmen der biologischen Abbaus freigesetzt. Es tritt also bei diesen bioresorbierbaren Medizinprodukten ein Auflösungsprozess ein, wobei die noch verbleibenden sich in der Auflösung befindenden Reste des Medizinprodukts weiterhin durch ein magnetisches Wechselfeld erwärmt werden können.

Entscheidend ist jedoch bei den erfindungsgemäßen Medizinprodukten, dass diese flexibel oder verformbar sind und dem Oberflächenverlauf eines Gewebes oder Organs oder des Operationsraumes nach einer chirurgischen Tumorentfernung folgen können. Somit sind die erfindungsgemäßen Medizinprodukte in Form von Geweben, weiche sich auf Gewebe oder Organe oder den Operationsbereich auflegen lassen und problemlos den unebenen Oberflächen folgen,oder in der Form
einer filmbildenen Zusammensetzung, welche sich naturgemäß auf jede unebene Fläche auftragen lässt.

In diesem Zusammenhang ist als Operationsbereich derjenige Bereich zu verstehen, der durch die äußeren Ränder einer Operationswunde abgegrenzt wird. Anders beschrieben ist der Operationsbereich der Übergangsbereich oder die Grenzfläche von Tumorgewebe zu gesundem Gewebe. Eine Behandlung bzw. Nachbehandlung dieses Bereiches ist sehr wichtig, um die Rezidivbildung zu verhindern.

Die hierin beschriebenen Medizinprodukte werden auf den Operationsbereich aufgebracht, aufgetragen und im Falle eines Sprays auf den Operationsbereich gesprüht und sind somit für eine Nachbehandlung der Operationswunde nach einer Tumoroperation gedacht.

Die erfindungsgemäßen Medizinprodukte sind somit vorrangig nicht für die systemische Anwendung gedacht, sondern für eine Implantation im Operationsbereich. Da die erfindungsgemäßen Medizinprodukte in der Regel vorzugsweise für die Dauer der nachfolgenden Chemotherapie im Operationsbereich verbleiben sollen, sind die erfindungsgemäßen Medizinprodukte je nach dem Zeitintervall geplanter Therapiesitzungen biologisch abbaubar, über einen längeren Zeitraum bioresorbierbar oder nicht abbaubar.

Wichtig ist, dass die erfindungsgemäßen Medizinprodukte, vorzugsweise biologisch abbaubaren oder langsam biologisch abbaubaren Medizinprodukte keine starre Form haben, sondern sich flexibel der abzudeckenden Oberfläche des Operationsbereichs anpassen können. Somit sind insbesondere flexible, leicht verformbare, sich leicht anderen Formen anpassbare oder formlose Medizinprodukte oder Träger für die aufheizbaren oder erwärmbaren Partikel bevorzugt.

Die erfindungsgemäßen Medizinprodukte sind somit sämtliche nicht starren und nicht metallischen Träger, weiche sich einer vorgegebenen Oberfläche anpassen und diese weitestgehend abdecken und zudem zur Aufnahme von magnetischen Partikeln, insbesondere superparamagnetischen Nanopartikeln geeignet sind. Die erfindungsgemäßen vorzugsweise biologisch abbaubaren Medizinprodukte sind medizinischer Zellstoff, Verbandsmaterialien, Wundeinlagen, chirurgisches Nahtmaterial, Kompressen, Schwämme oder medizinische Textilien.

Der medizinische Zellstoff und die medizinischen Textilien stellen vorzugsweise zweidimensionale Strukturen mit geringer Dicke dar, welche mit den Partikeln imprägniert sind. Die magnetischen Partikel lagern sich an die Faserstrukturen dieses Medizinproduktes an, welches in trockener oder vorbefeuchteter Form dann nach der Operation in die Wunde über den Operationsbereich gelegt wird.

Eine andere Form von erfindungsgemäßen Medizinprodukten sind Schwämme oder allgemein biologisch abbaubare poröse dreidimensionale Strukturen, welche die magnetischen Partikel sowohl auf der Oberfläche als auch im Inneren der porösen Struktur in den Hohlräumen als auch dem Schwammmaterial selbst enthalten können. Diese Schwämme werden nach einer Operation in die Wunde gelegt und füllen den Operationsraum weitgehend oder auch nur teilweise aus. Aus diesen schwammartigen Strukturen können die magnetischen Partikel freigesetzt werden, wobei die Partikel aber auch in fest gebundener Form vorliegen können. Die Freisetzung kann sowohl durch Diffusion von nur locker gebundenen Partikeln aus den Hohlräumen der porösen Struktur als auch durch biologischen Abbau der Schwammstruktur erfolgen, wenn die Partikel im Material der Schwammstruktur selbst eingearbeitet oder eingelagert sind.

Die erfindungsgemäßen Medizinprodukte sind für die Implantation in den menschlichen und tierischen Körper bestimmt und müssen daher physiologisch verträglich sein. Wichtig ist, dass die erfindungsgemäßen Medizinprodukte nicht in flüssiger Form als Lösung oder Suspension vorliegen, sondern in einer Formulierung, welche viskos oder zähflüssig oder filmbildend oder fest ist, damit das Medizinprodukt nach der Implantation auch an der gewünschten Stelle verbleibt.

Wichtig ist auch, dass sich das Medizinprodukt an beliebige Oberflächen anpasst, d.h. dem Oberflächenverlauf folgt.

Als "Medizinprodukt" wird hierin ein Träger mit den magnetischen Partikeln bezeichnet und als "Träger" dienen die hierin ausführlich beschriebenen Gewebe, Zellstoffe, filmbildende Zusammensetzungen, usw., welche biologisch abbaubar oder biostabil sein können und nicht magnetisch sind und daher ohne die magnetischen Partikel auch nicht in einem magnetischen Wechselfeld erwärmt werden können. Die Träger sind nicht aus lebender Materie, könnten Röntgenmarken oder Kontrastmittel enthalten und binden die Partikel vorzugsweise adhäsiv und/oder kovalent. Die Partikel sind hingegen zumeist nicht biologisch abbaubar, setzen Wärme bei Anregung durch ein Magnetwechselfeld frei und erwärmen dadurch nicht nur sich selbst, sondern auch den Träger, also das gesamte Medizinprodukt und dadurch auch das umliegende Gewebe. Ferner lassen sich wie weiter unten beschrieben, auch pharmakologische Wirkstoffe wie z.B. Zytostatika in das Medizinprodukt einbringen, welche durch Diffusion und/oder biologischen Abbau des Trägers und/oder die Wärmeentwicklung und/oder das magnetische Wechselfeld freigesetzt werden können, um vor allem Tumorzellen zu bekämpfen.

Als "Gewebe" wird hierin jedes medizinisch verwendete Textil oder Zellstoff bezeichnet, woraus Verbandsmaterialien, Wundauflagen, Verbände oder andere medizinische Tücher oder Stoffe hergestellt werden.

Der Begriff "biologisch abbaubares Medizinprodukt" bezieht auf ausdrücklich nur auf die Matrix für die magnetischen Partikel, nicht jedoch auf die magnetischen Partikel selbst, welche in der Regel nicht biologisch abbaubar sind. Biologisch abbaubar sind daher der medizinische Zellstoff, Verbandsmaterialien, Wundeinlagen, chirurgisches Nahtmaterial, Kompressen, Schwämme
oder medizinische Textilien, worin oder worauf die magnetischen Partikel aufgebracht oder eingebracht werden. Bei den erfindungsgemäßen abbaubaren Medizinprodukten mit den magnetischen Partikeln wird somit die Matrix für die magnetischen Partikel, d.h. das Medizinprodukt ohne die magnetischen Partikel biologisch abgebaut und die magnetischen Partikel bleiben in der Regel zurück oder reichern sich im Tumorgewebe bzw. in den Krebszellen an und werden zumeist nicht biologisch abgebaut oder nur ein Teil deren Beschichtung wird biologisch abgebaut, wobei der magnetische Kern in der Regel nicht biologisch abbaubar ist.

Als Operationsraum wird der Bereich verstanden, wo sich der entfernte Tumor oder das entfernte Krebsgewebe befunden hat.

Eine weitere bevorzugte Variante von Medizinprodukten sind flüssige oder gelförmige Formulierungen in Form von Salben, Cremes, Gelen und Sprays, insbesondere filmbildenden Sprays. Diese Formulierungen enthalten die magnetischen Partikel und werden nach Entfernung des Tumors auf den Operationsbereich aufgetragen oder aufgesprüht.

Die erfindungsgemäßen Medizinprodukte sind abgesehen von den magnetischen Partikeln vorzugsweise biologisch abbaubar und lösen sich daher vorzugsweise innerhalb von 1 bis 12 Monaten weiter bevorzugt 1 bis 6 Monaten vollständig auf, wobei auch die enthaltenen magnetischen Partikel freigesetzt werden.

Die Funktionsweise der erfindungsgemäßen Medizinprodukte besteht darin, dass diese den Operationsbereich möglichst vollständig abdecken sollten, damit die magnetischen Partikel möglichst nahe zu den noch verbliebenen Krebszellen oder noch verbliebenem Krebsgewebe kommen. Die magnetischen Partikel und vorzugsweise superparamagnetischen Partikel können in einem magnetischen Wechselfeld erwärmt werden, wodurch sich durch eine Thermotherapie die noch verbliebenen Krebszellen abtöten lassen. Dabei erwärmen die im erfindungsgemäßen Medizinprodukt enthaltenen Magnetpartikel das Medizinprodukt insgesamt und die aus dem Medizinprodukt heraus diffundierten magnetischen Partikel erwärmen die Krebszellen, an welche sie sich anlagern oder in welche sie eindringen.

Diese thermotherapeutische Behandlung kann zudem eine herkömmliche Chemotherapie bzw. Strahlentherapie unterstützen, weil die thermotherapeutische Behandlung vergleichsweise wenig Nebenwirkungen verursacht und zeitgleich mit einer chemotherapeutischen Behandlung durchgeführt werden kann. Da die erfindungsgemäßen Medizinprodukte den Operationsbereich möglichst vollständig bedecken bzw. den Operationsraum möglichst vollständig ausfüllen sollten, haben die erfindungsgemäßen Medizinprodukte einen möglichst direkten Kontakt zu den noch verbliebenen Krebszellen und noch verbliebenem Krebsgewebe, welches durch die unmittelbare Nähe der magnetischen Partikel besonders effektiv abgetötet werden kann. Die thermotherapeutische Behandlung mittels der erfindungsgemäßen Medizinprodukte ist daher deutlich selektiver und schonender als die Chemotherapie und Strahlentherapie.

Bei einer bevorzugten Ausführungsform der vorliegenden Erfindung ist an die erwähnten magnetischen Partikel mindestens eine pharmakologisch wirksame Substanz vorzugsweise ein Antikrebsmittel gebunden. Beispiele für geeignete Antikrebsmittel sind: Actinomycine, Aminoglutethimid, Amsacrin, Anastrozol, Antagonisten von Purin- und Pyrimidin-Basen, Anthracycline, Aromatasehemmer, Asparaginase, Antiöstrogene, Bexaroten, Bleomycin, Buselerin, Busulfan, Camptothecin-Derivate, Capecitabin, Carboplatin, Carmustin, Chlorambucil, Cisplatin, Cladribin, Cyclophosphamid, Cytarabin (Cytosinarabinosid), alkylierende Cytostatika, Dacarbazin, Dactinomycin, Daunorubicin, Docetaxel, Doxorubicin (Adriamycin), Epirubicin, Estramustin, Etoposid, Exemestan, Fludarabin, Fluorouracil, Folsäureantagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Melphalan, Mercaptopurin, Methotrexat, Miltefosin, Mitomycine, Mitosehemmstoffe, Mitoxantron, Nimustine, Oxaliplatin, Paclitaxel, Pentostatin, Procarbazin, Tamoxifen, Temozolomid, Teniposid, Testolacton, Thiotepa, Tioguanin, Topoisomerase-Inhibitoren, Topotecan, Treosulfan, Tretinoin, Triptorelin, Trofosfamide, Vinblastin, Vincristin, Vindesin, Vinoreibin, zytostatisch wirksame Antibiotika.

Die Ablösung mindestens einer therapeutisch wirksamen Substanz von den Partikeln kann ferner durch ein magnetisches Wechselfeld bewirkt oder initiiert werden. Dadurch kann erreicht werden, dass unmittelbar im Operationsbereich die thermotherapeutische Behandlung noch durch einen antiproliferativen Wirkstoff unterstützt wird, was die Effektivität abermals steigert. Natürlich ist auch hier eine gleichzeitige oder zeitversetzte zusätzliche Chemotherapie oder Strahlentherapie möglich.

Der mindestens eine pharmakologische Wirkstoff muss jedoch nicht zwingend an die Partikel vorzugsweise Nanopartikel gebunden werden. Er kann ohne Anbindung an die Partikel zusätzlich in dem erfindungsgemäßen Medizinprodukt enthalten oder auf dessen Oberfläche aufgebracht sein.

Die Anbindung des Wirkstoffs an die Partikel hat jedoch den Vorteil, dass eine eher zielgerichtete Freisetzung erfolgt, da der Wirkstoff zusammen mit dem Partikel in Krebszellen eindringen kann oder an Krebszellen angelagert wird und dort durch ein magnetisches Feld initiiert freigesetzt werden kann.

In diesem Zusammenhang bedeutet "durch ein magnetisches Wechselfeld bewirkt oder initiiert", dass zum einen das magnetische Wechselfeld bzw. die Impulse direkt die Freisetzung bzw. Ablösung bewirken oder indirekt beispielsweise über die Aktivierung von Enzymen oder die Erzeugung von Wärme eine Ablösung des Wirkstoffs erfolgt.

Die Nanopartikel enthaltenden Medizinprodukte in Form von medizinischem Zellstoff, Verbandsmaterialien, Wundeinlagen, chirurgisches Nahtmaterial, Kompressen, medizinische Schwämme oder medizinische Textilien
können somit noch mindestens einen pharmakologischen Wirkstoff vorzugsweise mindestens ein Antikrebsmittel enthalten. Geeignete Wirkstoffe sowie deren Anbindung an die Partikel werden weiter unten eingehend beschrieben.

Nach der Auftragung der Medizinprodukte bzw. der biologisch abbaubaren Medizinprodukte auf den Operationsbereich werden durch Anlegen eines äußeren magnetischen Wechselfeldes die erwähnten Implantate und implantierbare Medizinprodukte im magnetischen Wechselfeld erwärmt.

Die Erwärmung der Partikel erfolgt in einem alternierenden magnetischen Wechselfeld, wobei die Stärke des magnetischen Wechselfeldes bevorzugt zwischen 1 und 25 kA/m, weiter bevorzugt zwischen 2 und 18 kA/m und die Frequenz bevorzugt zwischen 5 bis 5.000 kHz weiter bevorzugt zwischen 10 und 1000 kHz liegen.

Unterstützt durch die Erwärmung werden die magnetischen Partikel vorzugsweise superparamagnetische Nanopartikel sowie die optional vorhandenen Wirkstoffe freigesetzt, welche sich an Krebszellen anlagern und diese abtöten. Diese eben beschriebene schonende Therapieform der Thermothermie ist insbesondere in Kombination mit anderen Behandlungsmethoden wie Strahlentherapie und/oder Chemotherapie einsetzbar.

### Magnetische Partikel

Erfindungsgemäß können beliebige magnetische Partikel eingesetzt werden, solange diese durch ein magnetisches Wechselfeld erwärmt werden können.

Bevorzugt sind demnach Mikropartikel und insbesondere Nanopartikel und insbesondere superparamagnetische Mikro- und Nanopartikel.

Die erwähnten Nanopartikel haben bevorzugt einen magnetischen, besonders bevorzugt einen superparamagnetischen Kern. Bevorzugt sind Materialien wie Maghämit, Magnetit, Eisen-Nickel-Legierungen, Nickel-Kupfer-Legierungen oder Kobalt-Nickel-Legierungen wie zum Beispiel FeNi oder CoNi.

Um die magnetischen Eigenschaften zu verbessern, kann auch eine zweite magnetische Kernschicht angebracht werden. Das Resultat ist ein höheres Gesamtkoerzitivfeld im Vergleich zu Nanopartikeln mit einschichtigem Kern. Die erste Kernschicht kann aus superparamagnetischem Material und die zweite Kernschicht aus einem von der ersten Kernschicht sich unterscheidenden Material bestehen. Um diesen Kern können weitere Schichten, die beispielsweise auch Wirkstoffe tragen, angebracht sein. Mehrschalige Partikel zur Einschleusung von Partikel-Wirkstoff-Konjugaten in Tumorzellen werden in der Anmeldung WO 98/58673 A beschrieben.

Der oder die Kerne selbst bestehen aus einem magnetischen Material, vorzugsweise einem ferromagnetischen, antiferromagnetischen, ferrimagnetischen, antiferrimagnetischen oder superparamagnetischen Material, weiter bevorzugt aus Eisenoxiden, insbesondere superparamagnetischen Eisenoxiden oder aus reinem Eisen, welches mit einer Oxidschicht versehen ist. Derartige Nanopartikel können durch ein magnetisches Wechselfeld mit einer bevorzugten Magnetfeldstärke zwischen 2 und 25 kA/m und einer Frequenz, die bevorzugt zwischen 5 und 5000 kHz liegt, erwärmt werden. Mit dieser Technik ist eine Erwärmung des die Nanopartikel enthaltenden Gewebes auf über 50°C möglich. Derartig hohe Temperaturen können erreicht werden, da bis zu 800 pg und mehr Eisen in Form der Nanopartikel pro Tumorzelle aufgenommen werden können und so die Nanopartikel über einen längeren Zeitraum die Zielregion nicht verlassen können und auf diese Weise sehr präzise und kontaktlos von außen, auch wiederholt Wärme im Tumor deponiert werden kann. Die Erwärmung beruht auf der Freisetzung von Translations- und Rotationswärme als Folge magnetischer Relaxationsvorgänge sowie Hysteresewärmeverlusten.

Die Nanopartikel bestehen vorzugsweise aus Eisenoxiden und insbesondere aus Magnetit (Fe₃O₄), Maghemit (γ-Fe₂O₃) oder Mischungen dieser beiden Oxide. Allgemein können die bevorzugten Nanopartikel durch die Formen FeO_{X} wiedergegeben werden, worin X eine rationale Zahl von 1 bis 2 bedeutet. Die Nanopartikel weisen vorzugsweise einen Durchmesser von weniger als 500 nm auf. Vorzugsweise besitzen die Nanopartikel einen durchschnittlichen Durchmesser von 15 nm oder liegen vorzugsweise in dem Größenbereich von 1 - 200 nm und insbesondere bevorzugt im Bereich von 5 - 30 nm.

Die Herstellung von Nanopartikeln ohne Wirkstoff und auch ohne Beschichtung ist ausführlich in DE 4428851 A beschrieben.

Neben den magnetischen Materialien der Formel FeO_{X}, worin X eine rationale Zahl im Bereich von 1,0 bis 2,0 ist, sind erfindungsgemäß auch Materialien der allgemeinen Formel MFe₂O₄ mit M = Co, Ni, Mn, Zn, Cd, Ba oder andere Ferrite einsetzbar.

Es ist auch möglich, Nanopartikel mit einem anderen als einem Eisenoxid-Metallkern auszustatten. Hierbei sind besonders die Metalle Gold, Silber, Platin, Kupfer, Kobalt, Nickel, Eisen, Mangan, Samarium, Neodym, Iridium, Osmium, Ruthenium, Rhodium, Palladium oder Legierungen der oben genannten Metalle zu nennen.

Es besteht aber auch die Möglichkeit, die Nanopartikel aus einem nicht magnetischen Material herzustellen, wie beispielsweise Siliziumdioxid (SiO₂). Ferner eignen sich auch Silica- oder Polymerpartikel, in die magnetische Materialien wie beispielsweise die oben genannten magnetischen Materialien eingelagert und/oder angebunden sind.

Die magnetischen Partikel können ferner dahingehend derivatisiert sein, dass sich chemische Strukturen wie z.B. Antikörper, Nukleinsäuren, Peptide, Aptamere oder andere Moleküle mit Zielfindungseigenschaften auf der Oberfläche der Partikel, befinden, welche die Affinität der Partikel zu entarteten Zellen steigern. Derartige Oberflächenmodifikationen verbessern die Affinität zu Krebszellen aufgrund Erkennung bestimmter Oberflächenstrukturen auf den entarteten Zellen. Bevorzugte chemische Strukturen, welche den magnetischen Partikeln Zielfindungseigenschaften verleihen, sind beispielsweise polyklonale Antikörper, monoklonale Antikörper, humanisierte Antikörper, humane Antikörper, chimäre Antikörper, rekombinante Antikörper, bispezifische Antikörper, Antikörperfragmente, Aptamere, Fab-Fragmente, Fc-Fragmente, Peptide, Peptidomimetika, gap-Mere, Ribozyme, CpG-Oligomere, DNA-Zyme, Riboswitches sowie Lipide.

Bei einer bevorzugten Ausführungsform der vorliegenden Erfindung können an die Nanopartikel optional therapeutisch wirksame Substanzen gebunden sein. Die Anbindung des Wirkstoffs kann kovalent oder durch eine überwiegend kovalente Bindung und/oder durch eine ausreichend starke ionische Bindung, Einlagerungsverbindung oder Komplexbindung erfolgen, so dass eine unkontrollierte Freisetzung des Wirkstoffs weitgehend unterbleibt. Als unkontrollierte Freisetzung wird die Ablösung des Wirkstoffs ohne die Einwirkung eines magnetischen Wechselfeldes verstanden.

Als therapeutisch wirksame Substanzen können anti-proliferative, anti-migrative, anti-angiogene, anti-thrombotische, antiinflammatorische, antiphlogistische, zytostatische, zytotoxische, anti-koagulative, anti-bakterielle, anti-virale und/oder anti-mykotische Wirkstoffe gewählt werden, wobei anti-proliferative, anti-migrative, anti-angiogene, zytostatische und/oder zytotoxische Wirkstoffe sowie Nukleinsäuren, Aminosäuren, Peptide, Proteine, Kohlenhydrate, Lipide, Glycoproteine, Glycane oder Lipoproteine mit anti-proliferativen, anti-migrativen, anti-angiogenen, anti-thrombotischen, antiinflammatorischen, antiphlogistischen, zytostatischen, zytotoxischen, anti-koagulativen, anti-bakteriellen, anti-viralen und/oder anti-mykotischen eigenschaften bevorzugt sein. Darüber hinaus können diese Substanzen auch Radiosensitizer oder Sensitizer oder Verstärker anderer auch kombinierter konventioneller Krebsbehandlungsmethoden sein oder solche Sentitizer enthalten.

Als zytotoxische und/oder zytostatische Verbindungen, d.h. chemische Verbindungen mit zytotoxischen und/oder zytostatischen Eigenschaften können unter anderem Alkylierungsmittel, Antibiotika mit zytostatischen Eigenschaften, Antimetabolite, Mikrotubuli-Inhibitoren und Topoisomerase-Inhibitoren, Platin-enthaltende Verbindungen und andere Zytostatika wie beispielsweise Asparaginase, Tretinoin, Alkaloide, Podophyllotoxine, Taxane und Miltefosin^{®}, Hormone, Immunmodulatoren, monoklonale Antikörper, Signaltransduktoren (Signaltransduktionsmoleküle) und Zytokine eingesetzt werden.

Als Beispiele für Alkylierungsmittel können unter anderem Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin und Nimustin genannt werden.

Beispiele für Antibiotika mit zytostatischen Eigenschaften sind Daunorubicin, Doxorubicin (Adriamycin), Dactinomycin, Mitomycin C, Bleomycin, Epirubicin (4-Epi-Adriamycin), Idarubicin, , Mitoxantron und Amsacrin.

Methotrexat, 5-Fluorouracil, 6-Thioguanin, 6-Mercaptopurin, Fludarabin, Cladribin, Pentostatin, Gemcitabin, Azathioprin, Raltitrexed, Capecitabin, Cytosinarabinosid, Thioguanin und Mercaptopurin können als Beispiele für Antimetabolite (antimetabolische Wirkstoffe) angeführt werden.

Zu der Klasse der Alkaloide und Podophyllotoxine gehören unter anderem Vincristin, Vinblastin, Vindesin, Etoposid als auch Teniposid. Des Weiteren können Platin-enthaltende Verbindungen erfindungsgemäß eingesetzt werden. Als Platin-enthaltende Verbindungen seien beispielsweise Cisplatin, Carboplatin und Oxaliplatin genannt. Zu den Mikrotubuli-Inhibitoren zählen beispielsweise Alkaloide wie beispielsweise Vinca-Alkaloide (Vincristin, Vinblastin, Vindesin, Venorelbin) und Taxane (Paclitaxel/Taxol^{®}, Paclitaxel und Docetaxel) sowie Derivate des Paclitaxel. Als Topoisomerase-Inhibitoren können beispielsweise Podophyllotoxine (Etoposid, Teniposid) und Camptotheca-Alkaloide (Camptothecin, Topotecan und Irinotecan) genannt werden.

Zu den anderen zytostatischen Wirkstoffen (anderen Zytostatika) zählen beispielsweise Hydroxycarbamide (Hydroxyurea), Imatinib, Miltefosin^{®}, Amsacrin, Pentostatin, Bexaroten, Tretinoin und Asparaginase. Vertreter der Verbindungsklasse der monoklonalen Antikörper sind unter anderem Trastuzumab (auch bekannt als Herceptin^{®}), Alemtuzumab (auch bekannt als MabCampath^{®}) und Rituximab (auch bekannt als MabThera^{®}).

Erfindungsgemäß können auch Hormone wie beispielsweise Glucocorticoide (Prednison), Oestrogene (Fosfestrol, Estramustin), LHRH (Buserelin, Goserelin, Leuprorelin, Triptorelin), Flutamid, Cyproteronacetat, Tamoxifen, Toremifen, Aminoglutethimid, Formestan, Exemestan, Letrozol und Anastrozol eingesetzt werden. Zu den Klassen der Immunmodulatoren, Zytokine, Antikörper und Signaltransduktoren zählen Interleukin-2, Interferon-α, Interferon-γ, Erythropoietin, G-CSF, Trastuzumab (Herceptin^{®}), Rituximab (MabThera^{®}), Efitinib (Iressa^{®}), Ibritumomab (Zevalin^{®}), Evevamisol sowie Retinoide.

Die vorgenannten Wirkstoffe können zusammen mit den magnetischen Teilchen im erfindungsgemäßen Medizinprodukt enthalten oder auf dessen Oberfläche aufgebracht sein. Für den Fall, dass der Wirkstoff an die magnetischen Partikel oder an das Medizinprodukt bzw. das biologisch abbaubare Medizinprodukt kovalent oder ionisch gebunden ist, findet die Anbindung des Wirkstoffs beispielsweise über Hydroxygruppen, Aminogruppen, Carbonylgruppen, Thiolgruppen oder Carboxylgruppen statt, je nachdem, welche funktionelle Gruppen der jeweilige Wirkstoff trägt.

Hydroxygruppen werden vorzugsweise als Ester, Acetal oder Kefial gebunden, Thiogruppen vorzugsweise als Thioester, Thioacetal oder Thioketal, Aminogruppen vorzugsweise als Amide und teilweise auch als Imine (Schiffsche Basen), Carboxylgruppen vorzugsweise als Ester oder Amide und Carbonylgruppen vorzugsweise als Ketale.

Zudem ist es bevorzugt, den oder die Wirkstoffe nicht direkt an ein Nanopartikel oder das Medizinprodukt bzw. das biologisch abbaubare Medizinprodukte zu binden, sondern über ein Linker-Molekül zu immobilisieren. Ferner ist die Funktionalisierung der Oberfläche der Nanopartikel bekannt, so dass nach bekannten Verfahren Aminogruppen, Hydroxygruppen, Carboxylgruppen oder Carbonylgruppen auf der Oberfläche der Nanopartikel erzeugt werden können.

Die therapeutisch wirksamen Substanzen werden direkt oder über ein Linker-Molekül vorzugsweise mittels einer Amidbindung oder Esterbindung an die Nanopartikel und/oder das Medizinprodukt bzw. das biologisch abbaubare Medizinprodukt gebunden.

Bevorzugt sind Linker, welche pH-spaltbare Acetal-, Ester-, Hydrazon- oder Imin-Gruppen enthalten und sauer oder mittels enzymatischer Reaktion gespalten werden können.

Als enzymatisch spaltbare Gruppe im oder am Linker-Molekül ist die Amidgruppe zu nennen. Thermisch oder mittels Säure spaltbare Gruppen umfassen z.B. Phosphatgruppen, Thiophosphatgruppen, Sulfatgruppen, Phosphamidgruppen, Carbamatgruppen oder Iminogruppen.

Der Wirkstoff muss nicht notwendigerweise kovalent an den Linker oder das bioresorbierbare Medizinprodukt gebunden sein, sondern kann auch ionisch oder über Wasserstoffbrücken gebunden oder interkaliert oder komplexiert vorliegen.

Wie bereits ausgeführt, können beliebige magnetische Partikel in den erfindungsgemäßen Medizinprodukten eingesetzt werden. Beispiele für derartige magnetische Partikel sind in WO 2005070471 A2, WO 0243708 A2, US 5,411,730 A1, WO 2005042142 A2, WO 03026618 A1, WO 2005065282 A2, WO 2006108405 A2 und WO 2007019845 A2 beschrieben.

### Biologisch abbaubare Medizinprodukte

Die erfindungsgemäßen biologisch abbaubaren Medizinprodukte in Form von Implantaten, Gewebe, Textil, Wundauflagen oder filmbildenden Zusammensetzungen verbleiben nach dem Verschließen der Wunde nach einer Krebsoperation durch den Chirurgen im Körper des Patienten.

Die erfindungsgemäßen biologisch abbaubaren Medizinprodukte dienen insbesondere zur Nachbehandlung des Operationsbereiches mit mittels Thermotherapie erzeugter Wärme zur Abtötung verbliebender Tumorzellen und zur Verhinderung der Rezidivbildung.

Somit bestehen die erfindungsgemäßen biologisch abbaubaren Medizinprodukte aus physiologisch verträglichen Materialien und/oder werden in physiologisch verträgliche Abbauprodukte und Bestandteile gespalten.

Die Materialien für die erfindungsgemäßen Medizinprodukte werden ausgewählt aus der Gruppe umfassend oder bestehend aus: Polyacrylsäure, Polyacrylate, Polymethylmethacrylat, Polybutylmethacrylat, Polyisobutylmethacrylat, Polyacrylamid, Polyacrylnitril, Polyamid, Polyetheramid, Polyethylenamin, Polyimid, Polycarbonat, Polycarbourethan, Polyvinylketon, Polyvinylhalogenid, Polyvinylidenhalogenid, Polyvinylether, Polyvinylaromaten, Polyvinylester, Polyvinylpyrollidon, Polyoxymethylen, Polyethylen, Polypropylen, Polytetrafluorethylen, Polyurethan, Polyolefin-Elastomer, Polyisobutylen, EPDM-Gummis, Fluorosilicon, Carboxymethylchitosan, Polyethylenterephtalat, Polyvalerat, Carboxymethylcellulose, Cellulose, Rayon, Rayontriacetat, Cellulosenitrat, Celluloseacetat, Hydroxyethylcellulose, Cellulosebutyrat, Celluloseacetat-butyrat, Ethylvinylacetat-copolymer, Polysulfon, Polyethersulfon, Epoxyharz, ABS-Harze, EPDM-Gummis, Siliconpräpolymer, Silicon, Polysiloxan, Polyvinylhalogen, Celluloseether, Cellulosetriacetat, Chitosan, Chitosanderivate, polymerisierbare Öle, Polyvalerolactone, Poly-ε-Decalacton, Polylactid, Polyglycolid, Copolymeren der Polylactide und Polyglycolide, Poly-ε-caprolacton, Polyhydroxybuttersäure, Polyhydroxybutyrat, Polyhydroxyvalerat, Polyhydroxybutyrate-co-valerat, Poly(1,4-dioxan-2,3-dion), Poly(1,3-dioxan-2-on), Poly-para-dioxanon, Polyanhydrid, Polymaleinsäureanhydrid, Polyhydroxymethacrylat, Polycyanoacrylat, Polycaprolactondimethylacrylat, Poly-ß-Maleinsäure Polycaprolactonbutyl-acrylat, Multiblockpolymeren aus Oligocaprolactondiol und Oligodioxanondiol, Polyetherester-multiblockpolymeren aus PEG und Poly(butylenterephtalat), Polypivotolacton, Polyglycolsäuretrimethyl-carbonat, Polycaprolacton-glycolid, Poly(γ-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonat, Polytrimethylcarbonat, Polyiminocarbonat, Polyvinylalkoholen, Polyesteramiden, glycolierten Polyestern, Polyphosphoestern, Potyphosphazenen, Poly[p-carboxy-phenoxy)propan], Polyhydroxypentansäure, Polyethylenoxid-propylenoxid, weichen Polyurethanen, Polyurethanen mit Aminosäureresten im Backbone, Polyetherester, Polyethylenoxid, Polyalkenoxalaten, Polyorthoestern, Carrageenanen, Stärke, Kollagen, Protein-basierten Polymeren, Polyaminosäuren, synthetische Polyaminosäuren, Zein, modifiziertes Zein, Polyhydroxyalkanoaten, Pectinsäure, Actinsäure, Fibrin, modifiziertes Fibrin, Casein, modifiziertes Casein, Carboxymethylsulfat, Albumin, Hyaluronsäure, Heparansulfat, Heparin, Chondroitinsulfat, Dextran, Cyclodextrine, Copolymeren aus PEG und Polypropylenglycol, Gummi arabicum, Guar, oder anderen Gummiharzen Gelatine, Collagen, Collagen-N-Hydroxysuccinimid, Lipiden, Lipoiden, polymersierbaren Ölen und deren Modifikationen, Copolymeren und Mischungen der vorgenannten Substanzen.

Die vorgenannten Polymere sind biologisch abbaubar oder können in Polymerisationsgraden und Vernetzungsgraden hergestellt werden, welche biologisch abbaubar sind.

Unter dem Begriff "biologisch abbaubar" oder "bioresorbierbar" wird verstanden, dass diese Materialien unter physiologischen Bedingungen innerhalb eines Zeitraums von 1 Monat bis zu 12 Monaten, vorzugsweise bis zu 6 Monaten zu über 90 Gew.-% abgebaut oder degradiert sind.

Bevorzugte bioabbaubare Polymere sind Polylactide, Polyglycolide, Copolymere der Polylactide und Polyglycolide, Polyhydroxybutyrate, Polyhydroxymethacrylate, Polyorthoester, glycolierte Polyester, Polyvinylalkohole, Polyvinylpyrrolidon, Acrylamid-Acrylsäure-copolymere, Hyaluronsäure, Heparansulfat, Heparin, Chondroitinsulfat, Dextran, β-Cyclodextrine, hydrophil vernetzte Dextrine, Alginate, Phospholipide, Carbomere, vernetzte Peptide und Proteine, Silikone, Polyethylenglycol (PEG), Polypropylenglycol (PPG), Copolymere aus PEG und PPG, Collagen, polymersierbare Öle und Wachse, wie deren Mischungen und Copolymere.

Des weiteren sind Polyester, Polylactide sowie Copoymere aus Diolen und Estern bzw. Diolen und Lactiden bevorzugt. Als Diole werden beispielsweise Ethan-1,2-diol, Propan-1,3-diol oder Butan-1,4-diol eingesetzt.

Erfindungsgemäß finden insbesondere Polyester für die polymere Schicht Verwendung. Aus der Gruppe der Polyester sind wiederum solche Polymere bevorzugt, welche die folgende Wiederholungseinheit besitzen:

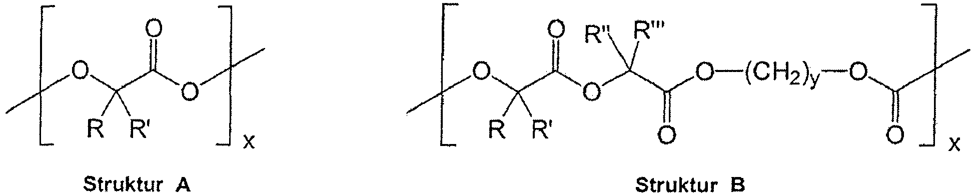

In den gezeigten Wiederholungseinheiten bedeutet R, R', R" und R'" einen Alkylrest mit 1 bis 5 Kohlenstoffatomen, insbesondere Methyl, Ethyl, Propyl, Isopropyl, n-Butyl, s-Butyl, t-Butyl, iso-Butyl, n-Pentyl oder Cyclopentyl und bevorzugt Methyl oder Ethyl. Y steht für eine ganze Zahl von 1 bis 9 und X steht für den Polymerisationsgrad. Insbesondere bevorzugt sind die folgenden Polymere mit den gezeigten Wiederholungseinheiten:

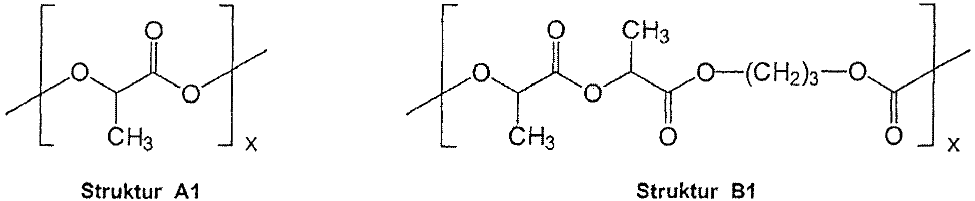

Als weitere Vertreter der resorbierbaren Polymere Resomer^{®} seien genannt die Poly(L-lactid)e mit der allgemeinen Formel -(C₆H₈O₄)ₙ- wie L 210, L 210 S, L 207 S, L 209 S, die Poly(L-lactid-co-D,L-lactid)e mit der allgemeinen Formel -(C₆H₈O₄)ₙ- wie LR 706, LR 708, L 214 S, LR 704, die Poly(L-lactid-co-trimethylcarbonat)e mit der allgemeinen Formel -[(C₆H₈O₄)ₓ-(C₄H₆O₃)_{y}]ₙ- wie LT 706, die Poly(L-lactid-co-glycolid)e mit der allgemeinen Formel -[(C₆H₈O₄)ₓ-(C₄H₄O₄)_{y}]ₙ- wie LG 824, LG 857, die Poly(L-lactid-co-ε-caprotacton)e mit der allgemeinen Formel -[(C₆H₈O₄)ₓ-(C₆H₁₀O₂)_{y}]ₙ- wie LC 703, die Poly(D,L-lactid-co-glycolid)e mit der allgemeinen Formel-[(C₆H₈O₄)ₓ-(C₄H₄O₄)_{y}]ₙ- wie RG 509 S, RG 502 H, RG 503 H, RG 504 H, RG 502, RG 503, RG 504, die Poly(D,L-lactid)e mit der allgemeinen Formel -[(C₆H₈O₄)ₙ- wie R 202 S, R 202 H, R 203 S und R 203 H. Resomer^{®} 203 S stellt hierbei den Nachfolger des insbesondere bevorzugten Polymers Resomer^{®} R 203 dar. Der Name Resomer^{®} repräsentiert ein hochtechnologisches Produkt der Firma Boehringer Ingelheim.

Grundsätzlich ist die Verwendung von resorbierbaren Polymeren bei der vorliegenden Erfindung besonders bevorzugt. Ferner sind Homopolymere der Milchsäure (Polylactide) sowie Polymere bevorzugt, die aus Milch- und Glykolsäure hergestellt werden.

### Biologisch stabile Medizinprodukte

Die erfindungsgemäßen biostabilen oder biologisch nicht abbaubaren Medizinprodukte in Form von Schwämmen, und insbesondere filmbildenden Zusammensetzungen, oder Textilien, Gewebe, Zellstoff, Wundauflagen und dergleichen, werden aus biologisch nicht oder kaum abbaubaren Materialien hergestellt.

Die Materialien für die erfindungsgemäßen biostabilen Medizinprodukte werden ausgewählt aus der Gruppe umfassend oder bestehend aus: Polyacrylsäure und Polyacrylate wie Polymethylmethacrylat, Polybutylmethacrylat, Polyacrylamid, Polyacrylonitrile, Polyamide, Polyetheramide, Polyethylertamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Epoxyharze, ABS-Harze, EPDM-Gummis, Silicone wie Polysiloxane, Polyvinylhalogene und Copolymere, Celluloseether, Cellulosetriacetate, Chitosan und Copolymere und/oder Mischungen derselben.

Bevorzugte biostabile Polymere, welche in der Medizintechnik und für biostabilen Implantate eingesetzt werden sind Polyethersulfon, substituiertes Polyethersulfon, Polyphenylsulfon, substituiertes Polyphenylsulfon, Polysulfonblockcopolymere, perfluorierte Polysulfonblockcopolymere, semifluorierte Polysulfonblockcopolymere, substituierte Polysulfonblockcopolymere und/oder Mischungen der vorgenannten Polymere.

### Gele

Die Nanopartikel können auch in Gele oder Hydrogele eingebracht werden oder Bestandteile von filmbildenden Sprays sein, welche vorzugsweise auch biologisch abbaubar sind. Zur besseren Stabilisierung der Gele oder filmbildenden Sprays können die hierin beschriebenen Nanopartikel mit Gel- oder Filmbildnern kombiniert werden.

Geeignete Gelbildner oder Filmbildner sind vorzugsweise Stoffe auf Cellulosebasis wie Cellulosenitrat oder Ethylcellulose oder physiologisch unbedenkliche Polymerisate davon, Polyvinylacetat, partiell verseiftes Polyvinylacetat, Mischpolymerisate aus Vinylacetat und Acrylsäure oder Crotonsäure oder Maleinsäuremonoalkylester, ternäre Mischpolymerisate aus Vinylacetat und Crotonsäure und Vinylneodecanoat, oder Crotonsäure und Vinylpropionat, Mischpolymerisate aus Methylvinylether und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, insbesondere mit einem Gehalt an quartären Ammoniumgruppen, oder Polymere, Copolymere oder Mischungen, enthaltend Ethylacrylat, Methylmethacrylat oder Trimethylammonioethylmethacrylat-chlorid, oder Polyvinylacetale und Polyvinylbutyrale, alkylsubstituierte Poly-N-vinylpyrrolidone, Alkylester aus Mischpolymerisaten aus Olefinen und Maleinsäureanhydrid, Umsetzungsprodukte von Kolophonium mit Acrylsäure sowie Benzoeharze, Chitosan, Luvimer 100^{®}, Alumniumstearat, Carbomere, Cocamid MEA, Carboxymethyldextran, Carboxymethylhydroxypropyl-Guar oder Rotalgen-Carrageenane.

In den vorgenannten Estern sind die Alkylreste gewöhnlich kurzkettig und haben meistens nicht mehr als vier C-Atome. Derartige Stoffe werden hierin auch als polymerbildende oder gelbildende Substanzen bezeichnet.

Zu den Gelbildnern bzw. Filmbildnern zählen zudem auch wasserlösliche Polymere wie beispielweise ionische Polyamide, Polyurethane und Polyester sowie Homo- und Copolymere von ethylenisch ungesättigten Monomeren. Derartige Stoffe sind zum Beispiel unter den Handelsnamen Acronal^{®}, Acudyne^{®}, Amerhold^{®}, Amphome^{®}, Eastman AQ^{®}, Ladival^{®}, Lovocryl^{®}, Luviflex VBM^{®}, Luvimer^{®}, Luviset P. U. R.^{®}, Luviskol^{®}, Luviskol Plus^{®}, Stepanhold^{®}, Ultrahold^{®}, Ultrahold Strong^{®} oder Versatyl^{®}. Bei Luvimer^{®} handelt es sich um ein Polyacrylat.

Weitere Komponenten der Gele können vor allem natürliche Polymere sein. Dazu zählen Albumin, Collagen, Hyaluronan, Chitosan und Chitin. Ein besonders bevorzugtes nicht natürliches Polymer ist Copolymer bzw. Blockcopolymer aus Polyethylenoxid mit endständigen α-Hydroxysäuren oder Poly-α-hydroxysäuren.

Des weiteren sind Glykosaminoglykane wie beispielsweise Aggrekan, Dekorin, Biglykan und Fibromodulin gängige Bestandteile der bioresorbierbaren Gele oder filmbildenden Lösungen oder Sprays.

Auch Salzlösungen wie beispielsweise physiologische (0,9-prozentige) Kochsalzlösung, PBS (phosphate buffered saline, d.h. Phosphat-gepufferte physiologische Kochsalzlösung) DMEM (Dulbecco's Modified Eagle Medium) können in den Gelen, Lösungen und Sprays eingesetzt werden.

Bei einer Verwendung von superparamagnetischen Nanopartikeln mit einem Eisenoxid-Kern ist ein Anteil von 3-30 Gew.-% Eisenoxid auf 200 mg Gel bevorzugt, weiter bevorzugt ein Anteil von 5-25 Gew.-% Eisenoxid auf 200 mg Gel und insbesondere bevorzugt ein Anteil von 10-20 Gew.-% Eisenoxid auf 200 mg Gel.

### Polymere Träger

Die magnetischen Partikel können bereits während der Herstellung von Polymeren zugesetzt werden und werden dann in die bioresorbierbare polymere Struktur eingebaut.

Beispiele für erfindungsgemäße biologisch abbaubare Medizinprodukte sind polymere Kügelchen enthaltend die magnetischen Partikel. Die Polymerkügelchen bestehen bevorzugt aus Polyhydroxybutyrat, Polylactid, Polyglykolid oder Copolymeren aus Polylactid-co-glykolid. Ein weiteres besonders bevorzugtes Material ist Alginat als auch Eudragit^{®}. Diese Polymerkügelchen enthalten bis zu 20 Gew.-% an magnetischen Partikeln.

Die Polymerkügelchen können als solche eingesetzt oder in Pasten eingearbeitet oder an medizinischen Zellstoff immobilisiert werden.

Die Polymerkügelchen können im magnetischen Wechselfeld bis zu Temperaturen von 50°C erwärmt werden.

### Medizinischer Zellstoff

Die beschichteten medizinischen implantierbaren Produkte, auf die die Nanopartikel aufgebracht werden, sind vorzugsweise bioresorbierbar. Dass heißt, sie können sich im Körper vollständig auflösen oder sind zumindest physiologisch gut verträglich.

Die Nanopartikel enthaltenden medizinischen Implantate sind unter anderem medizinischer Zellstoff, Verbandsmaterialien, Wundeinlagen, chirurgisches Nahtmaterial, Kompressen und medizinische Textilien.

Polyhydroxybutyrate und Cellulosederivate, Chitosanderivate als auch Collagen, Polyethylenglykol, Polyethylenoxid und Polylactide sind bevorzugte Materialien für die medizinischen Zellstoffe und Textilien. Werden Alginate als Wundauflage verwendet, werden bevorzugt Calciumalginat mit Natriumcarboxymethylcellulose verwobene Produkte verwendet. Hierbei ist das SeaSorb Soft der Firma Coloplast als Beispiel zu nennen.

Falls die Nanopartikel auf Wundverbände und/oder Wundeinlagen aufgebracht werden, sind besonders die Produkte Tabotamp^{®} und Spongostan^{®} der Firma Johnson und Johnson zu erwähnen. Diese Produkte werden durch kontrollierte Oxidation aus regenerierter Cellulose hergestellt.
Falls chirurgisches Nahtmaterial mit den Nanopartikel imprägniert werden soll, werden Nahtmaterialien verwendet, die bevorzugt aus Polyglykolsäure, Polycaprolacton-Coglykolid, oder aus Poly-p-dioxanon bestehen. Als Beispiele seien hier die Produkte Marlin^{®}, PCL und Marisorb^{®} der Firma Catgut GmbH genannt.

Falls Kompressen mit den Nanopartikel imprägniert werden sollen, sind hier besonders sterile Mullkompressen aus 100 % Baumwolle zu verwenden. Als Beispiele seien hier die Produktlinien Stericomp^{®} und Askina^{®} genannt.

Falls medizinischer Zellstoff verwendet wird, ist ein solcher, der einen Anteil von mehr als 90% Zellulose besitzt, bevorzugt.

Falls medizinische Textilien verwendet werden, sind Trevira^{®}-Produkte bevorzugt.

Die medizinischen Textilien und Zellstoffe werden mit einer Lösung der magnetischen Partikeln in Wasser, Ethanol oder Wasser-Ethanol-Gemischen besprüht oder darin getaucht, wobei der Tauch- oder Sprühvorgang nach dem Trocknen des Medizinproduktes mehrmals wiederholt werden kann.

Pro cm² Oberfläche des Medizinproduktes werden 10 µg bis 100 mg an magnetischen Partikeln aufgetragen.

Pro g des Medizinproduktes werden 100 µg bis 2 g an magnetischen Partikeln aufgetragen.

### Schwämme

Bei den medizinischen Schwämmen handelt es sich um bioresorbierbare Implantate mit schwammartiger, poröser Struktur.

Bevorzugte Materialien für die medizinischen Schwämme sind Collagen, oxidierte Cellulose, Chitosan, Thrombin, Fibrin, Chitin, Alginate, Hyaluronsäure, PLGA, PGA, PLA, Polysaccharide und Globin.

Falls medizinische Schwämme verwendet werden, sind solche, die einen Anteil von mehr als 90% Kollagen besitzen, bevorzugt.

Pro g des Medizinproduktes werden 100 µg bis 2 g an magnetischen Partikeln aufgetragen.

### Salben und Pasten

Falls die Nanopartikel in Salben eingebracht werden, wird eine Salbengrundlage verwendet bestehend aus gereinigtem Wasser in einer Menge von bevorzugt 5 - 50 Gew. %, besonders bevorzugt von 10 - 40 Gew. % und am meisten bevorzugt von 20 - 30 Gew. %. Die Salbe enthält zudem noch Vaseline in einer Menge von bevorzugt 40 - 90 Gew. %, besonders bevorzugt von 50 - 80 Gew. % und am meisten bevorzugt von 20 - 60 Gew. %. Zudem kann die Salbe noch dickflüssiges Paraffin in einer Menge von 5 - 50 Gew. %, besonders bevorzugt von 10 - 40 Gew. % und am meisten bevorzugt von 20 - 30 Gew. % enthalten.

Die hierin erwähnten Gelbildner und/oder Filmbildner können zudem in einer Menge von bis zu 30 Gew.-% zugesetzt werden. Zudem können Polymere wie beispielsweise Cellulose, Chitosan, Thrombin, Fibrinogen, Chitin, Alginate, Albumin, Hyaluronsäure, Hyaluronan, Polysaccharide, Globin, Polylactid, Polyglykolid, Polylactid-co-glykolid, Polyhydroxybutyrate, Cellulosederivate, Chitosanderivate, Polyethylenglykol und Polyethylenoxid in Mengen bis zu 30 Gew.-% eingesetzt werden.

### Filmbildende Sprays

Die erfindungsgemäßen Nanopartikel können auch in Sprühlösungen eingebracht werden oder Bestandteile von filmbildenden Sprays sein. Zur besseren Stabilisierung der filmbildenden Sprays können die hierin beschriebenen magnetischen Partikel oder Wirkstoff-enthaltende Nanopartikel mit Filmbildnern kombiniert werden. Filmbildende Sprays enthalten mindestens einen oder mehrere Filmbildner.

Geeignete Filmbildner sind vorzugsweise Stoffe auf Cellulosebasis wie Cellulosenitrat oder Ethylcellulose oder physiologisch unbedenkliche Polymerisate davon, Polyvinylacetat, partiell verseiftes Polyvinylacetat, Mischpolymerisate aus Vinylacetat und Acrylsäure oder Crotonsäure oder Maleinsäuremonoalkylester, ternäre Mischpolymerisate aus Vinylacetat und Crotonsäure und Vinylneodecanoat, oder Crotonsäure und Vinylpropionat, Mischpolymerisate aus Methylvinylether und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, insbesondere mit einem Gehalt an quartären Ammoniumgruppen, oder Polymere, Copolymere oder Mischungen, enthaltend Ethylacrylat, Methylmethacrylat oder Trimethylammonioethylmethacrylat-chlorid, oder Polyvinylacetale und Polyvinylbutyrale, alkylsubstituierte Poly-N-vinylpyrrolidone, Alkylester aus Mischpolymerisaten aus Olefinen und Maleinsäureanhydrid, Umsetzungsprodukte von Kolophonium mit Acrylsäure sowie Benzoeharze, Chitosan, Luvimer 100^{®}, Alumniumstearat, Carbomere, Cocamid MEA, Carboxymethyldextran, Carboxymethylhydroxypropyl-Guar oder Rotalgen-Carrageenane.

In den vorgenannten Estern sind die Alkylreste gewöhnlich kurzkettig und haben meistens nicht mehr als vier C-Atome.

Zu den Filmbildnern zählen auch wasserlösliche Polymere wie beispielweise ionische Polyamide, Polyurethane und Polyester sowie Homo- und Copolymere von ethylenisch ungesättigten Monomeren. Derartige Stoffe sind zum Beispiel unter den Handelsnamen Acronal^{®}, Acudyne^{®}, Amerhold^{®}, Amphome^{®}, Eastman AQ^{®}, Ladival^{®}, Lovocryl^{®}, Luviflex VBM^{®}, Luvimer^{®}, Luviset P. U. R.^{®}, Luviskol^{®}, Luviskol Plus^{®}, Stepanhold^{®}, Ultrahold^{®}, Ultrahold Strang^{®} oder Versatyl^{®}. Bei Luvimer^{®} handelt es sich um ein Polyacrylat als Hair Styling-Polymer entwickelt von der Firma BASF AG.

Als Lösungsmittel sind Wasser, Ethanol oder Wasser-Ethanol-Mischungen bevorzugt.

Bei einer Verwendung von superparamagnetischen Nanopartikeln mit einem Eisenoxid-Kern ist ein Anteil von 3-30 Gew.-% Eisenoxid auf 200 mg Gel bevorzugt, ein Anteil von 5-25 Gew.-% Eisenoxid auf 200 mg Gel besonders bevorzugt und ein Anteil von 10-20 Gew.-% Eisenoxid auf 200 mg Gel am meisten bevorzugt.

Pro g des Medizinproduktes werden 100 µg bis 2 g an magnetischen Partikeln aufgetragen.

Die Herstellung der Nanopartikel-enthaltenden Implantate erfolgt mittels Tauch- oder Sprühverfahren. Dabei werden die zu implantierenden Produkte in eine Nanopartikel-enthaltene Lösung oder Suspension eingetaucht oder mit einer Nanopartikel-enthaltenen Lösung besprüht. Danach werden die Produkte getrocknet und steril verpackt. Die Gele, Salben, Lösungen und Sprays werden erhalten, indem die gewünschte pharmazeutische Zubereitung nach Standardmethoden hergestellt wird und vorzugsweise in einem letzten Schritt die gewünschte Menge an magnetischen Partikeln zugesetzt wird.

Die erhaltenen biologisch abbaubaren Medizinprodukte werden zur Behandlung und Prophylaxe von Tumoren, Karzinomen und Krebs eingesetzt und dienen insbesondere zur Nachbehandlung des Operationsbereichs nach einer Krebsoperation und insbesondere nach Entfernung eines soliden Tumors.

Als Beispiele für Krebs- und Tumorarten, wo die erfindungsgemäßen Medizinprodukte eingesetzt werden können, sind zu nennen: Adenokarzinome, Aderhautmelanom, Akute Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hals-, Nasen- und Ohrentumoren, Hämatologische Neoplasien, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals-Nasen- und Ohrenbereichs), Kolonkarzinom, Kraniopharyngeome, Krebs im Mundbereich und auf der Lippe, Leberkrebs, Lebermetastasen, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakarzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome, u. osteoplastische Karzinome, Osteosarkom, Ovarial-Karzinom, Pankreaskarzinom, Peniskrebs, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs.

Bevorzugt sind insbesondere solide Tumoren. Ferner sind bevorzugt Prostatakarzinome, Gehirntumore, Sarkome, Zervix-Karzinome, Ovarialkarzinome, Mammakarzinome, Bronchialkarzinome, Melanome, Kopf-Hals Tumore, Ösophaguskarzinome, Rektumkarzinome, Pankreas-, Blasen- und Nierenkarzinome, Metastasen der Leber, des Gehirns und in Lymphknoten.

Insbesondere bevorzugt sind ferner die Anwendung und der Einsatz der erfindungsgemäßen bioresorbierbaren Medizinprodukte in der Medizin vorzugsweise zusammen mit der Strahlentherapie und/oder zusammen mit der herkömmlichen Chemotherapie.

Diese schonende Methode der Thermotherapie beinhaltet einen örtlich begrenzten Einsatz von Krebsmedikamenten und reduziert somit die Medikamentenlast und die Nebenwirkungen für den Patienten. Zudem wird die Wahrscheinlichkeit einer erneuten Metastasierung stark erniedrigt, da lokal eine Krebsbekämpfung der nach einer unvollständigen Resektion verbliebenen Tumorzellen selektiv stattfindet. Die eventuell sich auf dem erfindungsgemäßen Implantat oder dem erfindungsgemäßen Medizinprodukt befindlichen Medikamente können zudem durch ein von außen angelegtes magnetisches Wechselfeld vom Nanopartikel abgelöst werden und selektiver ihre Wirkung direkt am Wirkort entfalten. Dies erlaubt auch eine genauere Medikamentendosierung, da durch die lokale Therapieform keine Medikamente während eines Transports durch den Körper verloren gehen. Die oben beschriebene Methode ist auch mit Nanoteilchen ohne angebundenen Wirkstoff effektiv gegen Krebszellen durchführbar. Dabei lagern sich die Nanoteilchen an die Krebszellen an oder dringen in die Krebszellen ein und zerstören die Krebszellen durch ein von außen angelegtes Magnetfeld, welches die Magnetteilchen erwärmt.

Zur weiteren Affinitätssteigerung bezüglich bestimmter Zelltypen können auf der Oberfläche der Nanopartikel bzw. auf der äußeren Schicht oder Hülle der Nanopartikel noch Moleküle mit Zielfindungseigenschaften wie z.B. monoklonale Antikörper und/oder Aptamere gekoppelt werden.

In einer bevorzugten Ausführungsform der vorliegenden Erfindung bestehen die Kerne der magnetischen Nanopartikel aus Magnetit (Fe₃O₄), Maghemit (γ-Fe₂O₃) oder Mischungen dieser beiden Oxide und sind vorzugsweise superparamagnetisch. Die Kerne sind darüber hinaus durch kolloidale Schutzhüllen stabilisiert, die eine Anbindung der therapeutisch wirksamen Substanzen ermöglichen.

### Beispele

Beispiel 1 A:
Allgemeine Vorschrift zur Herstellung einer Nanopartikel-Suspension / Lösung für die Imprägnierung oder das Besprühen oder das Eintauchen der Träger
Eine Lösung von 0,23 Mol FeCl₂ und 0,46 Mol FeCl₃ in 1 I Wasser wird mit Stickstoff entgast. Darauf wird innerhalb von 20 Minuten soviel 5 M NaOH zugesetzt, dass ein pH-Wert von 11,5 erreicht wird. Der resultierende Niederschlag wird 10 Minuten auf 65°C erhitzt und anschließend innerhalb on 5 Minuten auf Raumtemperatur abgekühlt. Daraufhin wird der Niederschlag solange mit entionisiertem und entgastem Wasser suspendiert bis der pH-Wert der Waschlösung 9 erreicht hat. Der Niederschlag wird in Wasser suspendiert und die Suspension wird mit Eisessig auf pH 6 eingestellt. Zu der resultierenden Suspension werden 10 Vol.-% einer 30 gewichtsprozentigen wässrigen H₂O-Lösung gegeben und anschließend wird bis zur Beendigung der Gasentwicklung gerührt, worauf die Suspension mit Wasser auf einen Feststoffgehalt von 5 Gew.-% Eisenoxid verdünnt wird.

Beispiel 1 B (ohne Oxidation / mit Luftbegasung):
Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt. Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
   Die Partikel wurden 3mal mit einer Mischung aus Ethanol/Wasser gewaschen. Danach wurden die Partikel erneut in 900 ml Ethylenglykol suspendiert und mit Luftsauerstoff begast. Die Suspension wurde zum Siedepunkt des Ethylenglykols erhitzt und für 24 Stunden auf dieser Temperatur gehalten. Nach dem Abkühlen wurden die Partikel mit Wasser/Ethanol gewaschen und in Wasser suspendiert.
   Diese Partikel wurden analog zu Beispiel 1 G beschichtet.

Beispiel 1C (mit Oxidation / mit Luftbegasung):
Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt.
   Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
   Die Partikel wurden 3mal mit einer Mischung aus Ethanol/Wasser gewaschen. Danach wurden die Partikel erneut in 900 ml Ethylenglykol suspendiert und mit Luftsauerstoff begast. Die Suspension wurde zum Siedepunkt des Ethylenglykols erhitzt und für 24 Stunden auf dieser Temperatur gehalten.
   Nach dem Abkühlen wurden die Partikel mit Wasser/Ethanol gewaschen und in 900 ml 1M HNO₃ suspendiert. Dann wurden 450 ml einer 0,7M Eisennitrat-Lösung (Fe(NO₃)₃ * 9 H₂O) zugegeben und für eine Stunde unter Rückfluss gekocht (100°C). Die Partikel wurden 3 x mit je 500 ml Wasser gewaschen.
   Diese Partikel wurden analog zu Beispiel 1 G beschichtet.

Beispiel 1 D (ohne Oxidation / ohne Luftbegasung):
Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt. Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
   Die Partikel wurden 3mal mit einer Mischung aus Ethanol/Wasser gewaschen. Danach wurden die Partikel erneut in 900 ml Ethylenglykol suspendiert. Die Suspension wurde zum Siedepunkt des Ethylenglykols erhitzt und für 24 Stunden auf dieser Temperatur gehalten.
   Nach dem Abkühlen wurden die Partikel mit Wasser/Ethanol gewaschen und in Wasser suspendiert.

   Diese Partikel wurden analog zu Beispiel 1 G beschichtet.

Beispiel 1 E (mit Oxidation / ohne Luftbegasung):
Zur Herstellung von Eisenoxid-Nanopartikeln in Ethylenglykol wurden 0,1 mol FeCl₃*6H₂O und 0,2 mol FeCl₃ (wasserfrei), 50 g Natriumacetat und 195 g Diaminohexan in 900 ml Ethylenglykol gelöst und für 1 Stunde auf 60°C erhitzt.
   Dann wurde die Lösung innerhalb von 30 Minuten zum Siedepunkt erhitzt. Die Siedetemperatur wurde für 6 Stunden gehalten. Die entstandene Dispersion wurde langsam auf Raumtemperatur abgekühlt.
   Die Partikel wurden 3mal mit einer Mischung aus Ethanol/Wasser gewaschen.
   Danach wurden die Partikel erneut in 900 ml Ethylenglykol suspendiert. Die Suspension wurde zum Siedepunkt des Ethylenglykols erhitzt und für 24 Stunden auf dieser Temperatur gehalten.
   Nach dem Abkühlen wurden die Partikel mit Wasser/Ethanol gewaschen und in 900 ml 1 M HNO₃ suspendiert. Dann wurden 450 ml einer 0,7M Eisennitrat-Lösung (Fe(NO₃)₃ * 9 H₂O) zugegeben und für eine Stunde unter Rückfluss gekocht (100°C). Die Partikel wurden 3 x mit je 500 ml Wasser gewaschen. Diese Partikel wurden analog zu Beispiel 1G beschichtet.

Beispiel 1 F:
Zur Herstellung von Eisenoxid-Nanopartikeln wurde eine Lösung von 96 g Natriumhydroxid und 680 ml Ölsäure in 2000 ml Methanol zu einer Lösung aus 216 g Eisen(III)Chlorid-Hexahydrat in 500 ml Methanol gegeben. Der entstehende Feststoff wurde mit Methanol gewaschen und in Diethylether gelöst. Dann wurde mehrmals mit Wasser extrahiert. Der Feststoff wurde mit Aceton ausgefällt, gewaschen und im Vakuum getrocknet.
   75 g dieses Feststoffs wurden in 250 ml Trioctylamin gelöst und für 1 Stunde auf 120°C erhitzt.
   Dann wurde die Lösung in einem Autoklaven innerhalb von 30 Minuten auf 380°C erhitzt. Diese Temperatur wurde für 4 Stunden gehalten. Die entstandene Dispersion wurde langsam auf Raumtemperatur abgekühlt. Die Partikel wurden 3mal mit einer Mischung aus Ethanol/Wasser gewaschen.
   Danach wurden die Partikel in 300 ml Diethylenglykol-dibutylether suspendiert und mit Luftsauerstoff begast. Die Suspension wurde im Autoklaven auf 300°C erhitzt für 24 Stunden auf dieser Temperatur gehalten.
   Diese Partikel wurden wie in Beispiel 1C oxidiert und dann analog zu Beispiel 1G beschichtet.

Beispiel 1G:
Die Partikel aus Beispiel 1B bis 1F wurden bei hohen g-Zahlen abzentrifugiert und mit Ethanol gewaschen. 500 mg des gewaschenen Produktes wurden in eine Extraktionshülse (603g Firma Whatman) eingewogen und in eine Soxhletapparatur eingesetzt. 200 ml Ethanol als Extraktionsmittel wurden in den Vorlagekolben der Soxhletapparatur eingefüllt. Das Extraktionsmittel wurde zum Sieden erhitzt. Die kontinuierliche Extraktion wurde über 8 h durchgeführt und beinhaltete ca. 16 Extraktionszyklen. Dabei verfärbt sich die Ethanollösung gelblich. Nach Beendigung wurde die Extraktionshülse entnommen und das Pulver in ein Schlenkgefäß überführt und 1 h im Vakuum getrocknet.
   Zur Dispergierung der Partikel nach der Extraktion wurden 0,5 g des Nanopartikel-Pulvers aus Beispiel 4 in 20 ml 0,01 M HCl suspendiert. Dann wurden die Nanopartikel für 30 Minuten mit Ultraschall behandelt. Danach wurde 0,5 g festes Natriumoleat zugegeben.
   Zu 120 ml einer Mischung aus Wasser / Ethanol (3:1) und 1,5 Gew.% Ammoniak wurden 3,3 ml einer Partikel-Dispersion gemäß Beispiel 5 (0,97 mol/l Fe) und 2,14 ml Tetraethoxysilan gegeben. Die Dispersion wurde während der Zugabe gerührt und danach für 6 Stunden mit Ultraschall behandelt. Die Dispersion wurde durch Zentrifugation und Redispergierung in Wasser aufgereinigt.

Beispiel 2: Schwamm
Mit Nanopartikeln imprägnierte Wundeinlage
Ein handelsüblicher Tabotamp^{®}-Schwamm wurde für 6 Minuten in die unter Beispiel 1 hergestellte Nanopartikel-Suspension getaucht. Nach dem Trocknen wurde der Tauchvorgang noch weitere zwei Mal wiederholt. Alternativ kann die Suspension mit einer Spritze aufgetragen werden. Dieser Prozess kann mehrmals wiederholt werden, bis die gewünschte Beladung des Schwamms erreicht ist.
Beispiel 3: Medizinischer Zellstoff
Medizinischer Zellstoff beschichtet mit Nanopartikeln Ein 3 cm breites und 6 cm langes Stück einer Wundauflage wie beispielsweise SeaSorb Soft der Firma Coloplast bestehend aus Calciumalginat und Natriumcarboxymethylcellulose wurde 5 Mal mit ca. 1 ml der Nanopartikel-Suspension gemäß Beispiel 1 besprüht und nach jedem Sprühvorgang ca. 20 Minuten an der Luft getrocknet. Alternativ kann die Suspension mit einer Spritze aufgetragen werden. Dieser Prozess kann mehrmals wiederholt werden, bis die gewünschte Beladung des Schwamms erreicht ist.
Beispiel 4: Medizinischer Zellstoff mit Wirkstoff Medizinischer Zellstoff imprägniert mit Nanopartikeln und Zytostatikum Ein handelsüblicher medizinischer Zellstoff aus Natriumcarboxymethylcellulose, Poly-N-vinylpyrrolidone und Polyethylenoxid (5 cm²) wurde für 5 Minuten in eine nach Beispiel 1 hergestellte Nanopartikel-Suspension getaucht, welche 0,3 mg Paclitaxel pro ml Lösung enthielt. Nach dem Trocknen und Sterilisieren ist das Medizinprodukt einsatzbereit.
Beispiel 5: Gel
Herstellung eines erfindungsgemäßen Gels:
4 g einer Mischung von Collagen Typ I und Collagen Typ II werden in einem Liter einer 50 mM Essigsäurelösung gelöst. Die Collagenlösung wird für 45 Minuten bei 4°C und 9500 Umdrehungen pro Minute zentrifugiert. Der Überstand wird dekantiert, in einen Dialyseschlauch gefüllt und gegen 25 Liter einer 1M Essigsäurelösung zwei Tage dialysiert und danach weitere 4 Tage gegen Wasser dialysiert.
Danach wurde die Collagenlösung im Dialyseschlauch bis zu einer Konzentration von 20 mg/ml (2% w/v) aufkonzentriert.
Zur Herstellung des Gels wurden 10 ml der Collagenlösung mit 0,1 ml einer 1 N NaOH-Lösung und 1 ml DMEM (Dulbecco's Modified Eagle Medium 10 X) für eine Stunde bei 37°C inkubiert.
Danach wurde 1,5 g lyophilisierte Nanopartikel einer Größenverteilung von 1 - 100 nm zugesetzt.
Nach einer Operativen Entfernung eines soliden Dünndarmtumors wurde das Gel möglichst vollständig auf den Operationsbereich aufgetragen.
Eine nachfolgende Behandlung mittels Thermotherapie im magnetischen Wechselfeld zeigte eine Erwärmung der Operationsbereiche auf 53°C.

Beispiel 6: Gel mit Wirkstoff
Zu 10 g des gemäß Beispiel 5 hergestellten Gels werden 0,1 g des Zytostatikums Temozolomid gegeben und danach gut vermischt.
Die Verabreichung des Gels erfolgte wie in Beispiel 5 beschrieben.
Beispiel 7: Schwamm
2 g Globinpulver werden wie in US 2007031474 A beschrieben hergestellt.
Ein schwammartiges Implantat wird hergestellt, indem eine 1 %ige wässrige Suspension von oxidierter Cellulose bei pH 7,2 mit 1,5 Gew.-% Globinpulver lyophilisiert wird. Die oxidierte Cellulose kann auch in Form von Fasern oder zwei- oder dreidimensionalen Strukturen eingesetzt werden.
Die erhaltene schwammähnliche Struktur besteht aus ca. 100 mg oxidierter Cellulose und zwischen 40 und 200 mg Globin und hat ein Volumen von ca. 10 cm³ und eine Dicke von ca. 3 mm.
Die schwammartige Struktur wird mit Ethylenoxid sterilisiert und verpackt.
Beispiel 8A: Partikel mit Wirkstoff
Herstellung von Nanopartikeln mit angekoppeltem Mitomycin:
Zur Ankopplung des Zytostatikums Mitomycin an Aminosilan-stabilisierte Eisenoxid-Nanopartikel wird zunächst ein Konjugat aus Mitomycin und einem Aldehydfunktionalisierten Alkoxysilan (z.B. Triethoxysilylbutyraldehyd) synthetisiert. Auf diese Weise wird der Wirkstoff über eine Imin-Bindung angekoppelt. Dieses Konjugat wird unter Rühren zu einer wässrigen Dispersion Aminosilan-stabilisierter Partikel wie z.B. denen aus WO 97/38058 A gegeben. Zur Mischung wird Ethylenglykol gegeben und das Wasser destillativ entfernt. Dadurch wird das Konjugat aus Wirkstoff und Silan an die bereits vorhandene Hülle auf Aminosilan-Basis gekoppelt (kondensiert). Die Aufreinigung erfolgt durch Dialyse gegen Reinstwasser. Eine ausführliche Reaktionsbeschreibung ist in WO 2006108405 A2 enthalten.
Beispiel 8B:
Die Herstellung von Nanopartikeln mit über eine Avidinbrücke an das Partikel angebundenes Doxorubicin wird wie in WO 2006108405 A2 beschrieben durchgeführt.

Beispiel 8C:
Die Herstellung von Nanopartikeln mit über eine Nukleotidsequenz angekoppeltem Doxorubicin wird wie in WO 2006108405 A2 beschrieben durchgeführt.

Beispiel 9: Schwamm
Eine schwammartige Struktur wird wie in Beispiel 7 beschrieben hergestellt, wobei anstelle von oxidierter Cellulose ein Gemisch aus Collagen Typ I, Collagen Typ II und Chitosan (25 Gew.-% : 25 Gew.-% : 50 Gew.-%) verwendet wird.
Danach wird der erhaltene Schwamm mit einer wäßrigen Suspension von Nanopartikeln mit angekoppeltem Doxorubicin gemäß Beispiel 8B oder 8C getränkt und getrocknet. Anstelle des nachträglichen Tränkens kann die Nanopartikel-Suspension gemäß Beispiel 8A, 8B oder 8C auch Suspension entsprechend Beispiel 7 zugesetzt und zusammen mit den anderen Komponenten lyophilisiert werden.
Beispiel 10: Medizinischer Zellstoff Medizinischer Zellstoff auf Basis von Chitosan, Uronsäure und Carboxymethyldextran (4 cm² ca. 20 mg) wird flach in einer Petrischale ausgebreitet und mit einer wäßrigen Suspension enthaltend die Nanopartikel mit angekoppeltem Mitomycin gemäß Beispiel 8A beträufelt bis die Beladung des Zellstoffs mit 50 mg an Nanopartikeln erreicht wird.
Beispiel 11: Gel mit Nanopartikeln 23,5 Gew.-% nicht hydriertes Lecithin, 20,0 Gew.-% Propylenglykol, 10,0 Gew.-% Ethanol, 2,5 Gew.-% Sorbitol, 0,05 M Phosphatpuffer (ad 100,0%) wurden bei Raumtemperatur für 16 h gerührt.
Um ein Nanopartikel-Gel zu erhalten, wurde das so erhaltene Gel mit der Nanopartikel-Suspension aus Beispiel 1 für 4 h gerührt.
Beispiel 12: Filmbildendes Spray mit Nanopartikeln
172 g Maleinsäurediethylester (Zulauf 1), 98 g Maleinsäureanhydrid (Zulauf 2, in einem beheizbaren Tropftrichter), 200 g Vinylisobutylether (Zulauf 3) und 12 g tert-Butylper-neodecanoat (Zulauf 4) werden in entsprechende Dosiergefäße gegeben. In einem 2L-Rührbehälter, der mit Rührer, Heizung, Rückflußkühler und den vorbereiteten Dosiervorrichtungen, sowie mit Gasein- und -auslaß ausgestattet ist, werden 111 ml von Zulauf 1, 10 ml von Zulauf 3 und 3 ml von Zulauf 4 vorgelegt und auf 60°C erhitzt. Bei dieser Temperatur werden der restliche Zulauf 1, der restliche Zulauf 3 und der Zulauf 2 in 3 Stunden und der restliche Zulauf 4 in 4 Stunden zudosiert. Anschließend wird noch 1 Stunde bei 80°C gerührt. Man erhält eine farblose hochviskose Schmelze, die bei dieser Temperatur mit 18 g Wasser versetzt und 1 h gerührt wird. Nach Abkühlen auf 75°C werden 480 g Ethanol innerhalb von 15 Minuten zudosiert und 1 h bei dieser Temperatur gerührt. Nach Abkühlen auf 25°C erhält man eine klare, viskose Polymerlösung mit einem Feststoffgehalt von 48,1 Gew.-%.
Um ein filmbildendes Nanopartikel-Spray zu erhalten, wurde die so erhaltene viskose Polymerlösung mit der Nanopartikel-Suspension aus Beispiel 1 für 2 h gerührt.
Beispiel 13: Filmbildendes Spray mit Nanopartikeln und Wirkstoff 10 ml der gemäß Beispiel 12 erhaltenen Polymerlösung werden mit 100 mg Carboplatin und 1000 mg lyophilisierter Nanopartikel gemäß Beispiel 1 versetzt.
Beispiel 14: Behandlung von Cervix-, Brustwand- und HNO-Tumoren Auf einem Knochen wird ein mit einer Nanopartikellösung gemäß Beispiel 1A (2 Molar & 3 Molar) getränktes Trägermaterial aufgebraucht. Der Knochen wird im Therapiegerät positioniert und einem Magnetwechselfeld ausgesetzt. Bei einer möglichst konstanten Umgebungstemperatur wird die auf dem Knochen zu messende Temperaturerhöhung bestimmt. Dieser Versuchsaufbau zeigt, dass mittels nanopartikelbeschichteter Träger, welche auf einem oder im Bereich eines Knochens appliziert werden im magnetischen Wechselfeld Cervix-, Brustwand- und HNO-Tumoren behandelt werden können.
Materialien
- Geräte:
   - Therapiegerät MFH-12TS,
   - Umlaufkühler (Julabo; FC600S) mit Schlauchverbindungen,
   - Fixator (Ratten-) mit Schlauchanschlüssen,
   - Polytec Luxtron (Modell: LAB. KIT) mit 2 Temperaturmesssonden,
   - Messgerät für die Feldstärke (mit Sonde),
   - Wasserbäder (37°C),
   - Eichsonde (kalibriert bis 11/09)
- Material:
   - 2M oder 3M Nanopratikelsuspension gemäß Beispiel 1A: jeweils 15 Minuten beschallt
   - Trägermaterial:
      ▪ 1: SPONGOSTAN Puder
         (1g, Resorbierbares Gelantinepuder, hämostatisch; Johnson+Johnson)
      ▪ 2: SPONGOSTAN Special
         (7x5x0,1cm, Resorbierbarer hämostatischer Gelantineschwamm; Johnson+Johnson)
      ▪ 3: Gelita Tampon
         (1x1x1cm, schwammartiges, aus gehärteter Gelantine porcinen Ursprungs, Hämostyptikum, das biologisch vollständig abbaubar ist; B. Braun Melsungen AG)
      ▪ 4: Lyostypt
         (3x5cm, nassstabile Kompresse aus nativen Kollagen bovinen Ursprungs, zur lokalen Blutstillung, resorbierbar; B. Braun Melsungen AG)
   - Knochen ("Schweine- Rippchen"),
   - Pinzetten,
   - Knetmasse,
   - Medizinisches Pflaster, (Durapore™; 3M; 2,5cmx9,14m)
   - Kalt / Warm Kompresse (Pharma-Depot GmbH; 13x14cm)
   - Tuberkulinspritze (Omnifix^{®}-F; Braun; 0,01mL/1mL),
   - Einmalinjektionskanüle (Sterican®; Braun; 27Gx11/2", 0,40x40mm),
   - Messschieber (DialMax. Kalibriert bis 08/09; MS150-4/Atl),
   - Skalpell (Klinge Nr.11),
   - Bechergläser,
   - Fotoapparat,
- Chemikalien:
   - Wasserstoffperoxid (H₂O₂; 30%),
   - Algenat (Alginic acid sodium salt),
Versuchsaufbau ein geeigneter Fixator wurde mittels Umlaufkühler auf 55°C temperiert und Dichtigkeitstests wurden durchgeführt.
1. der Fixator wurde im Spalt des Therapiegerätes positioniert,
2. in den "Kopfbereich" des Fixators wurde eine vorgewärmte (37°C) kalt / warm Kompresse gelegt (verringerte den Luftraum im Gefäß und "puffert" die Temperaturschwankungen etwas ab),
3. Knochen:
▪ Ein Knochen wurde aus den Rippchen gelöst und grob vom Fleisch befreit,
▪ in ein Becherglas H₂O₂ gelegt,
▪ im Anschluss wurde der Knochen mit einem Skalpell "geputzt",
▪ der Knochen wurde mit einer Säge in versuchsgerechte Teile geteilt

4. Feldstärke:
▪ an der Position im Fixator, an der später die Messungen durchgeführt werden, wird der Messkopf für die Feldstärkenmessung positioniert, Knetmasse dient hierbei als Markierungshilfe
▪ Es werden 3 Feldstärken (für die klinische Anwendung relevant) ausgemessen: 3,0 kA/m, 3,5 kA/m, 4,0 kA/m
▪ diese Messungen ergaben folgende Werte:

| kA/m | %-Feldstärke |
|---|---|
| 3,03 | 14,5 |
| 3,49 | 17,0 |
| 4,07 | 20,5 |

% Feldstärke ist die Geräteeinstellung, welche der zugeordneten Feldstärke in kA/m entspricht
5. im Fixator wird Temperatur des Luftraumes und des Applikatorbodens bestimmt,
Beispiel 14A: Lyostypt
Partikel: Nanopratikelsuspension gemäß Beispiel 1A (0,5 mL, 2 Molar)
Träger: Lyostypt^{®}, Größe: (19,95x14,9x3,4) mm
Knochen: Größe: (44,4 x 13,2 x 10,9) mm
Ein Knochenteil wurde vermessen [Maße: (44,4x13,2x10,9) mm] und ein Stück Träger [Maße: (19,95x14,9x3,4) mm] wurde zugeschnitten. Der Träger wird auf dem Knochen platziert und mit den Partikeln getränkt (0,5 mL, 2 Molar nach Beispiel 1A). Der beladene Knochen wird im Applikator positioniert [Sonde 1 (rot): senkrecht von oben auf dem getränkten Träger; Sonde 2 (blau): Basalwert ("leerer" Knochen)] und für den Träger werden die Messwerte bestimmt:

| Feldstärke soll: 3,0kA/m → 14% | |
|---|---|
| 0:00:00 | Sonde1: ca.33°C, MF↑, Lüfter↑ |
| 2:00 | Sonde1: ca.36,5°C |
| 5:15 | Sonde1: ca.36,0°C, Trägersubstanz rutscht langsam unter der Sonde weg |
| 8:40 | Sonde1: ca.35,9°C |
| 9:40 | Sonde1↓ da "ausgezogen" |
| 10:00 | Sonde1: ca.35,8°C, MF↓ |
| 11:48 | Sonde1: 32,8°C, Sonde neu ausgerichtet |
| 12:30 | Sonde1: 32,5°C, MF↑ (14%) |
| 17:26 | Sonde1: 35,4°C, MF↓ |
| 20:44 | Neue Position: Sonde liegt zwischen Trägersubstanz und Knochen |
| 21:45 | Sonde1: 31,8°C, MF↑ (14%), Lüfter↑ |
| 26:47 | Sonde1: 33,7°C, MF↓ |
| 28:30 | Lüfter↓ |

| Feldstärke soll: 3,5kA/m → 17% (neues Stück Trägersubstanz mit gleichen Abmaßen und Volumen Nanotherm, Sonde zwischen Trägersubstanz und Knochen) | |
|---|---|
| 0:00:00 | Sonde1: 26,0°C, MF↑, Lüfter↑ |
| 2:00 | Sonde1: 30,3°C |
| 2:35 | Sonde1:31,0°C |
| 2:58 | Sonde1: 31,3°C |
| 3:26 | Sonde1: 31,6°C |
| 3:46 | Sonde1:31,9°C |
| 4:00 | Sonde1:32,0°C |
| 5:00 | Sonde1: 32,5°C |
| 5:44 | Sonde1: 32,8°C |
| 6:51 | Sonde1:33,0°C |
| 8:00 | Sonde1: 33,4°C |
| 9:00 | Sonde1: 33,5°C |
| 10:00 | Sonde1: 33,6°C, MF↓ |
| ca. 12:30 | Sonde1: 29,0°C |
| ca. 14:00 | Sonde1: 29,2°C |

| | |
|---|---|
| Feldstärke soll: 4,0kA/m → 20,5% (unveränderter Aufbau) | |
| 0:00:00 | Sonde1: 29,3°C, MF↑, Lüfter↑ |
| 1:00 | Sonde1: 33,0°C |
| 2:00 | Sonde1: 34,7°C (Raumluft zieht! Ursache?) |
| 3:15 | Sonde1: 35,4°C |
| 4:00 | Sonde1: 35,7°C |
| 5:15 | Sonde1: 35,9°C |
| 6:30 | Sonde1: 36,0°C |
| 8:00 | Sonde1: 36,0°C |
| 9:00 | Sonde1: 36,1°C |
| 9:15 | Sonde1: 36,0°C; Sonde2: 34,5°C; MF↓ |
| ca.12:00 | Sonde1: 30,5°C; Sonde2: 34,6°C; Lüfter↓ |

Die Messsonde ist immer zwischen dem Träger und dem Knochen positioniert.

| | |
|---|---|
| MF↑: | Magnetwechselfeld ein |
| MF↓: | Magnetwechselfeld aus |
| Lüfter↑: | Lüfter ein |
| Lüfter↓: | Lüfter aus |
| Sonde1↓: | Sonde funktioniert nicht |

Beispiel 14B: SPONGOSTAN

| | |
|---|---|
| Partikel: | Nanopratikelsuspension gemäß Beispiel 1A (1,5 mL, 2 Molar) |
| Träger: | Spongostan Puder, Masse: 0,3 g |
| Knochen: | Größe: (44,4 x 13,2 x 10,9) mm |

1,08 g Puder (Träger) wird mit 1,5 mL Partikel (2 Molar nach Beispiel 1A) getränkt und gut vermischt und eine Teilmenge von m = 0,46 g getränktem Träger wird auf dem Knochen modelliert.
An der Position im Fixator, an der später die Messungen durchgeführt werden, wird der Messkopf für die Feldstärkenmessung positioniert, Knetmasse dient hierbei als Markierungshilfe. Es werden 3 Feldstärken (für die klinische Anwendung relevant) ausgemessen: 3,0 kA/m, 3,5 kA/m, 4,0 kA/m

| Feldstärke soll: 3,0kA/m → 14% | |
|---|---|
| 0:00:00 | Sonde1: 33,6°C, MF↑, Lüfter↑ |
| 2:00 | Sonde1: 34,8°C |
| 10:00 | Sonde1: 35,7°C, MF↓ |
| nach 2' | Sonde1: 35,3°C |

| Feldstärke soll: 3,5kA/m → 17% | |
|---|---|
| 0:00:00 | Sonde1: 35,8°C, MF↑ (Lüfter an) |
| 2:00 | Sonde1: 36,0°C |
| 4:00 | Sonde1: 36,1 °C |
| 5:00 | Sonde1: 36,3°C, MF↓ |

| Feldstärke soll: 4,0kA/m → 20,05% | |
|---|---|
| 0:00:00 | Sonde1: 36,8°C, MF↑, Lüfter↑ |
| 7:50 | Sonde1: 39,0°C |
| 9:00 | Sonde1: 39,2°C |
| 10:00 | Sonde1: 39,3°C, MF↓ |
| nach 2' | Sonde1: 38,8°C |

Beispiel 14C: SPONGOSTAN

| | |
|---|---|
| Partikel: | Nanopratikelsuspension gemäß Beispiel 1A (1,6 mL, 2 Molar) |
| Träger: | getränkter Träger aus Beispiel 14B, Masse: ca. 0,8 g |
| Knochen: | Größe: (44,4 x 13,2 x 10,9) mm |

Der in Beispiel 14B übrig gebliebene Menge an getränktem Träger von ca. 0,8 g wird mit 1,6 ml Partikel (2 Molar gemäß Beispiel 1A) versetzt und auf dem gemäß Beispiel 14 gereinigten Knochen aufgetragen. Die Sonde wird wieder zwischen Knochen und Träger platziert. Es wurde wieder bei den 3 Feldstärken (3,0 kA/m, 3,5 kA/m, 4,0 kA/m) gemessen.

| Feldstärke soll: 3,0kA/m → 14% | |
|---|---|
| 0:00:00 | Sonde1: 24,1°C, MF↑, Lüfter↑ |
| 7:00 | Sonde1 :32,0°C |
| 10:00 | Sonde1: 33,2°C |
| 12:02 | Sonde1: 33,5°C. MF↓ |
| nach 4' | Sonde1: 30,8°C |

| Feldstärke soll: 3,5kA/m → 17% | |
|---|---|
| 0:00:00 | Sonde1: 30,7°C, MF↑ (Lüfter an) |
| 1:00 | Sonde1: 33,1°C |
| 5:30 | Sonde1: 35,6°C |
| 8:00 | Sonde1: 35,7°C |
| 10:00 | Sonde1: 36,1°C, MF↓ |
| nach 1' | Sonde1: 33,3°C |
| nach 2' | Sonde1: 32,2°C |
| nach 3' | Sonde1: 31,8°C |

| Feldstärke soll: 4,0kA/m → 20,05% | |
|---|---|
| 0:00:00 | Sonde1: 31,6,8°C, MF↑, Lüfter↑ |
| 1:00 | Sonde1: 34,7°C |
| 4:00 | Sonde1: 37,0°C |
| 5:00 | Sonde1: 37,4°C |
| 6:00 | Sonde1: 37,7°C |
| 7:00 | Sonde1: 37,6°C |
| 9:00 | Sonde1: 37,9°C |
| 10:00 | Sonde1: 38,2°C, MF↓ |
| nach 30" | Sonde1: 35,4°C |

Beispiel 14D: SPONGOSTAN Special

| | |
|---|---|
| Partikel: | Nanopratikelsuspension gemäß Beispiel 1A (1,0 mL, 2 Molar) |
| Träger: | getränkter Träger aus Beispiel 14B, Größe: (10,0x10,0x2,0)mm |
| Knochen: | Größe: (44,4 x 13,2 x 10,9) mm |

Damit der Träger die Partikel (1,0 mL, 2 Molar gemäß Beispiel 1A) aufnimmt, muss der Träger mit der Partikelsuspension getränkt (15 Minuten) werden. In eine Verpackung wird 1 mL Partikelsuspension zu dem Träger [m=0,00] gespritzt, der Quader nimmt das Maximum auf und wird auf den Knochen gelegt.
Die Messung erfolgt wie in Beispiel 14A beschrieben.

| Feldstärke soll: 3,0kA/m → 14% | |
|---|---|
| 0:00:00 | Sonde1: 25,5°C, MF↑, Lüfter↑ |
| 6:30 | Sonde1: 30,6°C |
| 8:00 | Sonde1: 31,1 °C |
| 9:00 | Sonde1: 31,4°C |
| 10:00 | Sonde1: 31,7°C |
| 11:00 | Sonde1:32,0°C |
| 12:00 | Sonde1:32,0°C |
| 12:30 | Sonde1: 32,0°C, MF↓ |
| nach 1'30" | Sonde1: 29,8°C, Lüfter↓ |
| nach 3' | Sonde1: 29,6°C |

| Feldstärke soll: 3,5kA/m → 17% | |
|---|---|
| 0:00:00 | Sonde1: 29,6°C, MF↑, Lüfter↑ |
| 1:00 | Sonde1:32,0°C |
| 5:00 | Sonde1: 34,1 °C |
| 10:00 | Sonde1: 34,6°C, MF↓ |
| nach 1' | Sonde1: 31,8°C |

| Feldstärke soll: 4,0kA/m → 20,05% | |
|---|---|
| 0:00:00 | Sonde1: 30,5°C, MF↑, Lüfter↑ |
| 2:30 | Sonde1: 35,5°C |
| 4:00 | Sonde1: 36,2°C |
| 5:00 | Sonde1: 36,6°C |
| 10:00 | Sonde1: 36,8°C, MF↓ |

Beispiel 14E: Gelita Tampon

| | |
|---|---|
| Partikel: | Nanopratikelsuspension gemäß Beispiel 1A (1,0 mL, 2 Molar) |
| Träger: | Gelita Tampon, Größe: (1x1x1) cm |
| Knochen: | Größe: (44,4 x 13,2 x 10,9) mm |

Damit der Träger die Nanopratikelsuspension (1,0 mL, 2 Molar gemäß Beispiel 1A) aufnimmt, muss der Träger mit der Partikelsuspension getränkt (15 Minuten) werden. In die Verpackung wird 1 mL Partikelsuspension zu dem Träger Gelita Tampon gespritzt, der Würfel nimmt das Maximum auf (Gelita Tampon [m=0,00] mit Nanopratikelsuspension: m=0,45g) und wird auf dem Knochen gelegt.

| Feldstärke soll: 3,0kA/m → 14% | |
|---|---|
| 0:00:00 | Sonde1: 29,1 °C, MF↑, Lüfter↑ |
| 5:00 | Sonder: 34,7°C |
| 7:00 | Sonde1: 35,3°C |
| 10:00 | Sonde1: 35,8°C |

| Feldstärke soll: 3,5kA/m → 17% | |
|---|---|
| 0:00:00 | Sonde1: 33,7°C, MF↑, (Lüfter an) |
| 1:00 | Sonde1: 35,2°C |
| 5:00 | Sonde1: 36,7°C |
| 10:00 | Sonde1: 37,1°C, MF↓ |

| Feldstärke soll: 4,0kA/m → 20,05% | |
|---|---|
| 0:00:00 | Sonde1: 34,9°C, MF↑, (Lüfter an) |
| 2:00 | Sonde1: 37,8°C |
| 3:00 | Sonde1: 38,3°C |
| 5:00 | Sonde1: 38,8°C |
| 7:00 | Sonde1: 39,0°C |
| 10:00 | Sonde1: 39,2°C, MF↓ |

## Patentansprüche

1. Ein durch ein magnetisches Wechselfeld erwärmbares, festes oder gelartiges Medizinprodukt zur Verwendung in der Nachbehandlung des operierten Bereichs bei Krebsoperationen, wobei das Medizinprodukt in Form eines physiologisch verträglichen Gewebes, Schwammes oder Films vorliegt, und wobei magnetische Partikel in dem Medizinprodukt enthalten sind, welche angeregt durch ein magnetisches Wechselfeld Wärme erzeugen und dadurch das Medizinprodukt erwärmen.

2. Medizinprodukt zur Verwendung nach Anspruch 1, wobei
i) die Partikel ortsfest in dem Medizinprodukt eingelagert oder angehaftet sind; und/oder
wobei ii) die Partikel dauerhaft in dem Medizinprodukt verbleiben, nicht durch Diffusion freigesetzt werden und nur im Falle von biologisch abbaubaren Medizin Produkten durch den Abbauprozess freigesetzt werden; und/oder
wobei iii) das Medizinprodukt verformbar ist und dem Oberflächenverlauf eines Gewebes oder Organs oder des Operationsraumes nach einer chirurgischen Tumorentfernung folgen kann.

3. Medizinprodukt zur Verwendung nach einem der vorherigen Ansprüche, wobei das Medizinprodukt biologisch abbaubar ist.

4. Medizinprodukt zur Verwendung nach einem der vorherigen Ansprüche, wobei es sich bei den Partikeln um Mikropartikel oder Nanopartikel handelt, und/oder wobei die Partikel paramagnetisch oder superparamagnetisch sind.

5. Medizinprodukt zur Verwendung nach einem der vorherigen Ansprüche, wobei
i) das Medizinprodukt mit den magnetischen Partikeln imprägniert, beschichtet oder getränkt ist, und/oder
ii) das Medizinprodukt innerhalb von 1 bis 12 Monaten biologisch resorbierbar ist, und/oder
iii) das Medizinprodukt physiologisch verträglich ist und in physiologisch verträgliche Bestandteile abgebaut wird, und/oder
iv) das Medizinprodukt flexibel ist und nicht aus Metall oder einer Metalllegierung besteht und wobei das Medizinprodukt nicht als wässrige Lösung der Partikel vorliegt.

6. Medizinprodukt zur Verwendung nach einem der Ansprüche 3 - 5, wobei das biologisch abbaubare Medizinprodukt die magnetischen Partikel freisetzt und/oder an umgebendes Tumorgewebe oder Tumorzellen abgibt.

7. Medizinprodukt zur Verwendung nach einem der vorherigen Ansprüche, wobei die magnetischen Partikel in einer Konzentration von 10 µg bis 100 mg pro cm² Oberfläche des Medizinproduktes enthalten sind, und/oder
wobei die magnetischen Partikel in einer Konzentration von 100 µg bis 2 g pro g des Medizinproduktes enthalten sind.

8. Medizinprodukt zur Verwendung nach einem der vorherigen Ansprüche, wobei es sich bei dem Medizinprodukt um medizinischen Zellstoff, Verbandsmaterialien, Wundeinlagen, chirurgisches Nahtmaterial, Kompressen, Schwämme, medizinische Textilien, oder filmbildende Zusammensetzungen handelt.

9. Medizinprodukt zur Verwendung nach einem der vorherigen Ansprüche, wobei das Medizinprodukt des Weiteren mindestens eine therapeutisch wirksame Substanz ausgewählt aus der Gruppe umfassend anti-proliferative, anti-migrative, anti-angiogene, anti-thrombotische, anti-inflammatorische, anti-phlogistische, zytostatische, zytotoxische, antikoagulative, anti-bakterielle, anti-virale und/oder anti-mykotische Wirkstoffe enthält, insbesondere wobei die mindestens eine therapeutische Substanz ausgewählt wird aus der Gruppe umfassend Actinomycin D, Aminoglutethimid, Amsacrin, Anastrozol, Antagonisten von Purin- und Pyrimidin-Basen, Anthracycline, Aromatasehemmer, Asparaginase, Antiöstrogene, Bexaroten, Bleomycin, Buselerin, Busulfan, Camptothecin-Derivate, Capecitabin, Carboplatin, Carmustin, Chlorambucil, Cisplatin, Cladribin, Cyclophosphamid, Cytarabin, Cytosinarabinosid, alkylierende Cytostatika, Dacarbazin, Dactinomycin, Daunorubicin, Docetaxel, Doxorubicin, Epirubicin, Estramustin, Etoposid, Exemestan, Fludarabin, Fluorouracil, Folsäurearitagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Melphalan, Mercaptopurin, Methotrexat, Miltefosin, Mitomycine, Mitosehemmstoffe, Mitoxantron, Nimustine, Oxaliplatin, Paclitaxel, Pentostatin, Procarbazin, Tamoxifen, Temozolomid, Teniposid, Testolacton, Thiotepa, Tioguanin, Topoisomerase-Inhibitoren, Topotecan, Treosulfan, Tretinoin, Triptorelin, Trofosfamide, Vinblastin, Vincristin, Vindesin, Vinorelbin, zytostatisch wirksame Antibiotika.

10. Medizinprodukt zur Verwendung nach Anspruch 9, wobei der mindestens eine Wirkstoff adhäsiv, ionisch, kovalent oder über einen Linker an das Partikel gebunden ist.

11. Medizinprodukt zur Verwendung nach einem der Ansprüche 9 oder 10, wobei die Ablösung des mindestens einen Wirkstoffes vom Träger über ein magnetisches Wechselfeld initiiert wird.

12. Medizinprodukt zur Verwendung nach einem der vorherigen Ansprüche, wobei das Medizinprodukt aus folgendem Material besteht: Polyacrylsäure, Polyacrylate, Polymethylmethacrylat, Polybutylmethacrylat, Polyisobutylmethacrylat, Polyacrylamid, Polyacrylnitril, Polyamid, Polyetheramid, Polyethylenamin, Polyimid, Polycarbonat, Polycarbourethan, Polyvinylketon, Polyvinylhalogenid, Polyvinylidenhalogenid, Polyvinylether, Polyvinylaromaten, Polyvinylester, Polyvinylpyrrolidon, Polyoxymethylen, Polyethylen, Polypropylen, Polytetrafluorethylen, Polyurethan, Polyolefin-Elastomer, Polyisobutylen, EPDM-Gummis, Fluorosilicon, Carboxymethylchitosan, Polyethylenterephthalat, Polyvalerat, Carboxymethylcellulose, Cellulose, Rayon, Rayontriacetat, Cellulosenitrat, Celluloseacetat, Hydroxyethylcellulose, Cellulosebutyrat, Celluloseacetatbutyrat, Ethylvinylacetatcopolymer, Polysulfon, Polyethersulfon, Epoxyharz, ABS-Harze, EPDM-Gummis, Siliconpräpolymer, Silicon, Polysiloxan, Polyvinylhalogen, Celluloseether, Cellulosetriacetat, Chitosan, Chitosanderivate, polymerisierbare Öle, Polyvalerolactone, Poly-ε-Decalacton, Polylactid, Polyglycolid, Copolymeren der Polylactide und Polyglycolide, Poly-ε-caprolacton, Polyhydroxybuttersäure, Polyhydroxybutyrat, Polyhydroxyvalerat, Polyhydroxybutyrat-co-valerat, Poly(1,4-dioxan-2,3-dion), Poly(1,3-dioxan-2-on), Polyparadioxanon, Polyanhydrid, Polymaleinsäureanhydrid, Polyhydroxymethacrylat, Polycyanoacrylat, Polycaprolactondimethylacrylat, Poly-ß-Mateinsäure, Polycaprolactonbutylacrylat, Multiblockpolymeren aus Oligocaprolactondiol und Oligodioxanondiol, Polyetherester-Multiblockpolymeren aus PEG und Poly(butylenterephthalat), Polypivotolacton, Polyglycolsäuretrimethylcarbonat, Polycaprolactonglycolid, Poly(γ-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonat, Polytrimethylcarbonat, Polyiminocarbonat, Polyvinylalkoholen, Polyesteramiden, glycolierten Polyestern, Polyphosphoestern, Polyphosphazenen, Poly [p-carboxyphenoxy)propan], Polyhydroxypentansäure, Polyethylenoxidpropylenoxid, weichen Polyurethanen, Polyurethanen mit Aminosäureresten im Backbone, Polyetherester, Polyethylenoxid, Polyalkenoxalaten, Polyorthoestern, Carrageenanen, Stärke, Kollagen, Protein-basierten Polymeren, Polyaminosäuren, synthetische Polyaminosäuren, Zein, modifiziertes Zein, Polyhydroxyalkanoaten, Pectinsäure, Actinsäure, Fibrin, modifiziertes Fibrin, Casein, modifiziertes Casein, Carboxymethylsulfat, Albumin, Hyaluronsäure, Heparansulfat, Heparin, Chondroitinsulfat, Dextran, Cyclodextrine, Copolymeren aus PEG und Polypropylenglycol, Gummi arabicum, Guar, oder anderen Gummiharzen, Gelatine, Collagen, Collagen-N-Hydroxysuccinimid, Lipiden, Lipoiden, polymersierbaren Ölen und deren Modifikationen, Copolymeren und Mischungen der vorgenannten Substanzen.

13. Medizinprodukt zur Verwendung nach einem der vorherigen Ansprüche, wobei der Krebs, Tumor oder die proliferative Erkrankung ausgewählt ist aus der Gruppe umfassend: Adenokarzinome, Aderhautmelanom, Akute Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, gastrointestinale Tumoren, Gallenblasenkrebs, Gallenkarzinome, Gebärmutterkrebs, Gebärmutterhalskrebs, Glioblastome, gynäkologische Tumoren, Hals-, Nasen- und Ohrentumoren, hämatologische Neoplasien, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals-Nasen- und Ohrenbereichs), Kolonkarzinom, Kraniopharyngeome, Krebs im Mundbereich und auf der Lippe, Leberkrebs, Lebermetastasen, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakanzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome, u. osteoplastische Karzinome, Osteosarkom, Ovarial-Karzinom, Pankreaskarzinom, Peniskrebs, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger-Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms-Tumor, Zervixkarzinom und Zungenkrebs.

14. Medizinprodukt zur Verwendung nach einem der vorherigen Ansprüche, wobei die Nachbehandlung des operativen Bereichs der Verhinderung von Rezidivbildung dient.

15. Verwendung eines durch ein magnetisches Wechselfeld erwärmbaren, festen oder gelartigen physiologischen Gewebes, Schwamms, oder Films zur Herstellung eines Medizinproduktes zur Nachbehandlung des operierten Bereichs bei Krebsoperationen, wobei magnetische Partikel in dem Medizinprodukt enthalten sind, welche angeregt durch ein magnetisches Wechselfeld Wärme erzeugen und dadurch das Medizinprodukt erwärmen.

## Claims

1. A solid or gel-like medical product heatable by an alternating magnetic field in the after-treatment of an operative field in cancer surgeries, wherein the medical product is present in the form of a physiologically acceptable tissue, sponge or film, and wherein magnetic particles are contained in the medical product which generate heat if excited by an alternating magnetic field and thereby heat the medical product.

2. Medical product for use according to claim 1, wherein
i) the particles are embedded into or attached to the medical product in a stationary position;
wherein ii) the particles remain permanently in the medical product, are not released by diffusion and are only released in case of biodegradable medical products due to the degradation process; and/or
wherein iii) the medical product is deformable and can follow the surface contours of a tissue or organ or the field of surgery after surgical tumor removal.

3. Medical product for use according to any one of the preceding claims, wherein the medical product is biodegradable.

4. Medical product for use according to any one of the preceding claims, wherein the particles are microparticles or nanoparticles, and/or wherein the particles are paramagnetic or superparamagnetic.

5. Medical product for use according to any one of the preceding claims, wherein
i) the medical product is impregnated, coated or soaked with the magnetic particles, and/or
ii) the medical product is bioresorbable between 1 to 12 months, and/or
iii) the medical product is physiologically acceptable and is degraded to physiologically acceptable components, and/or
iv) the medical product is flexible and does not consist of metal or a metal alloy, and wherein the medical product is not provided in the form of an aqueous solution of the particles.

6. Medical product for use according to any one of claims 3 - 5, wherein the biodegradable medical product releases the magnetic particles and/or delivers them to surrounding tumor tissue or tumor cells.

7. Medical product for use according to any one of the preceding claims, wherein the magnetic particles are present at a concentration of 10 µg to 100 mg per cm² of the surface of the medical product, and/or wherein the magnetic particles are present at a concentration of 100 µg to 2 g per g of the medical product.

8. Medical product for use according to any one of the preceding claims, wherein the medical product is medical cellulose, bandaging materials, wound inserts, surgical sutures, compresses, sponges, medical textiles, or film-forming compositions,

9. Medical product for use according to any one of the preceding claims, wherein the medical product further comprises at least one therapeutically effective agent selected from the group comprising anti-proliferative, anti-migratory, anti-angiogenic, anti-thrombotic, anti-inflammatory, anti-phlogistic, cytostatic, cytotoxic, anti-coagulative, anti-bacterial, anti-viral and/or anti-mycotic drugs, in particular wherein the at least one therapeutic substance is selected from the group comprising actinomycin D, aminoglutethimide, amsacrin, anastrozol, antagonists of purine or pyrimidine bases, anthracycline, aromatase inhibitors, asparaginase, anti-estrogens, bexaroten, bleomycin, buselerin, busulfan, camptothecin derivatives, capecitabin, carboplatin, carmustin, chlorambucil, cisplatin, cladribine, cyclophosphamide, cytarabine, cytosine arabinoside, alkylating cytostatics, dacarbazine, dactinomycin, daunorubicin, docetaxel, doxorubicin, epirubicin, estramustin, etoposide, exemestan, fludarabine, fluorouracil, folic acid antagonists, formestan, gemcitabine, glucocorticoids, goselerin, hormones and hormone antagonists, hycamtin, hydroxyl urea, idarubicin, ifosfamide, imatinib, irinotecan, letrozol, leuprorelin, lomustin, melphalan, mercaptopurine, methotrexate, miltefosin, mitomycin, mitosis inhibitors, mitoxantrone, nimustine, oxaliplatin, paclitaxel, pentostatin, procarbazine, tamoxifen, temozolomide, teniposide, testolacton, thiotepa, tioguanine, topoisomerase inhibitors, topotecan, treosulfan, tretinoin, triptorelin, trofosfamide, vinblastine, vincristine, vindesin, vinorelbin, cytostatically effective antibiotics.

10. Medical product for use according to claim 9, wherein the at least one drug is bound adhesively, ionically, covalently or via a linker to the particle.

11. Medical product for use according to any one od claims 9 or 10, wherein the detachment of the at least one drug from the carrier is initiated by an alternating magnetic field.

12. Medical product for use according to any one of the preceding claims, wherein the medical product consists of the following material: polyacrylic acid, polyacrylate, polymethyl methacrylate, polybutyl methacrylate, polyisobutyl methacrylate, polyacrylamide, polyacrylnitrile, polyamide, polyetheramide, polyethyleneamine, polyimide, polycarbonate, polycarbourethane, polyvinylketone, polyvinylhalogenide, polyvinylidenhalogenide, polyvinylether, polyvinyl aromatics, polyvinyl ester, polyvinylpyrolidone, polyoxymethylene, polyethylene, polypropylene, polytetrafluoroethylene, polyurethane, polyolefin elastomer, polyisobutylene, EPDM gums, fluorosilicone, carboxyxmethylchitosan, polyethyleneterephthalate, polyvalerate, carboxymethylcellulose, cellulose, rayon, rayon triacetate, cellulose nitrate, cellulose acetate, hydroxyethyl cellulose, cellulose butyrate, cellulose acetate-butyrate, ethylvinylacetate copolymer, polysulfone, polyethersulfone, epoxy resins, ABS resins, EPDM gums, silicone pre-polymer, silicone, polysiloxane, polyvinyl halogen, cellulose ether, cellulose triacetate, chitosan, chitosan derivatives, polymerizable oils, polyvalerolactone, poly-ε-decalacton, polylactide, polyglycolide, co-polymers of polylactide and polyglycolide, poly-ε-caprolactone, polyhydroxy butyric acid, polyhydroxybutyrate, polyhydroxyvalerate, polyhydroxybutyrate-co-valerate, poly(1,4-dioxan-2,3-dione), poly(1,3-dioxan-2-one), poly-para-dioxanone, polyanhydride, polymaleic acid anhydride, polyhydroxy methacrylate, polycyanoacrylate, polycaprolacton dimethylacrylate, poly-β-maleic acid, polycaprolactonbutyl acrylate, multi-block polymers made of oligocaprolactonediol and oligodioxanondiol, polyetherester multi-block polymers made of PEG and poly(butyleneterephthalate), polypivotolactone, polyglycolic acid trimethylcarbonate, polycaprolactone-glycolide, poly(γ-ethylglutamate), poly(DTH-iminocarbonate), poly(DTE-co-DT-carbonate), poly(bisphenol A-iminocarbonate), polyorthoester, polyglycolic acid trimethyl-carbonate, polytrimethylcarbonate, polyiminocarbonate, polyvinylic alcohols, polyester amides, glycolidized polyesters, polyphosphoesters, polyphosphazenes, poly[p-carboxyphenoxy)propane], polyhydroxypentanoic acid, polyethylene oxide-propylene oxide, soft polyurethanes, polyurethanes with amino acid residues in the backbone, polyether esters, polyethylene oxide, polyalkenoxalates, polyorthoesters, carrageenans, starch, collagen, protein-based polymers, polyamino acids, synthetic polyamino acids, zein, modified zein, polyhydroxyalkanoates, pectic acid, actinic acid, fibrin, modified fibrin, casein, modified casein, carboxymethylsulphate, albumin, hyaluronic acid, heparin sulphate, heparin, chondroitin sulphate, dextrane, cyclodextrine, co-polymers made of PEG and polypropyleneglycol, gum arabic, guar, or other gum resins, gelatin, collagen, collagen-n-hydroxysuccinimide, lipids, lipoids, polymerizable oils and their modifications, co-polymers and mixtures of the aforementioned substances.

13. Medical product for use according to any one of the preceding claims, wherein the cancer, tumor or the proliferative disease is selected from the group comprising: adenocarcinomas, choroidal melanoma, acute leukemia, acoustic neurinoma, ampullary carcinoma, anal carcinoma, astrocytomas, basalioma, pancreatic carcinoma, connective tissue tumor, bladder cancer, bronchial carcinoma, non-small cell bronchial carcinoma, breast cancer, Burkitt's lymphoma, corpus carcinoma, CUP syndrome, colon cancer, cancer of the small intestine, small intestine tumors, ovarian cancer, endometrial carcinoma, ependymoma, epithelial cancers, Ewing's sarcoma, gastrointestinal tumors, gallbladder cancer, biliary carcinomas, uterine cancer, cervical cancer, glioblastomas, gynecological tumors, otorhinolaryngologic tumors, hematologic neoplasias, urethral cancer, skin cancer, brain tumors (gliomas), brain metastases, testicular cancer, hypophyseal tumor, carcinoids, Kaposi's sarcoma, laryngeal cancer, germinal tumor, bone cancer, colorectal carcinoma, head-neck tumor (tumors of neck, nose and ear area), colon carcinoma, craniopharyngiomas, cancer in the mouth area and on the lips, hepatic cancer, hepatic metastases, eyelid tumor, lung cancer, lymphatic gland cancer (Hodgkin/Non-Hodgkin), lymphomas, stomach cancer, malignant melanoma, malignant neoplasma, malignomas of the gastrointestinal tract, mammary carcinoma, rectal cancer, medulloblastomas, melanoma, meningeomas, Hodgkin's disease, mycosis fungoides, nose cancer, neurinoma, neuroblastoma, kidney cancer, renal cell carcinoma, Non-Hodgkin's lymphomas, oligodendroglioma, esophageal carcinoma, osteolytic carcinoma and osteoplastic carcinoma, osteosarcoma, ovarian carcinoma, pancreatic carcinoma, penile cancer, squamous cell carcinomas of the head and neck, prostate cancer, pharyngeal cancer, rectal carcinoma, retinoblastoma, vaginal cancer, thyroid carcinoma, Schneeberger's disease, esophageal cancer, spinalioma, T-cell lymphoma (mycosis fungoides), thymoma, tubal carcinoma, eye tumors, urethral cancer, urologic tumors, urothelial carcinoma, vulvar cancer, mastoid involvement, soft tissue tumors, soft tissue sarcoma, Wilms' tumor, cervix carcinoma and tongue cancer.

14. Medical product for use according to any one of the preceding claims, wherein the after-treatment of the operative field serves for the prevention of relapse formation.

15. Use of a solid or gel-like physiological tissue, sponge or film heatable by an alternating magnetic field for the preparation of a medical product for after-treatment of an operative field in cancer surgeries, wherein magnetic particles are contained in the medical product, which generate heat if excited by an alternating magnetic field and thereby heat the medical product.

## Revendications

1. Dispositif médical solide ou en gel, pouvant être chauffé par un champ magnétique alternatif, pour utilisation lors du post-traitement de la zone opérée lors d'opérations du cancer, le dispositif médical se présentant sous forme d'un tissu, d'une éponge ou d'un film physiologiquement compatible, et des particules magnétiques étant contenues dans le dispositif médical, qui sont excitées par un champ magnétique alternatif, produisent de la chaleur et de ce fait chauffent le dispositif médical.

2. Dispositif médical pour l'utilisation selon la revendication 1, dans lequel
i) les particules sont incorporées à demeure dans le dispositif médical, ou y adhèrent à demeure ; et/ou
dans lequel ii) les particules restent en permanence dans le dispositif médical, ne sont pas libérées par diffusion, et ne sont libérées que dans le cas de dispositifs médicaux biodégradables, sous l'effet du processus de dégradation ; et/ou
dans lequel iii) le dispositif médical est déformable et peut suivre la surface d'un tissu ou d'un organe ou du champ opératoire après un enlèvement chirurgical d'une tumeur.

3. Dispositif médical pour l'utilisation selon l'une des revendications précédentes, le dispositif médical étant biodégradable.

4. Dispositif médical pour l'utilisation selon l'une des revendications précédentes, dans lequel, pour ce qui concerne les particules, il s'agit de microparticules ou de nanoparticules, et/ou dans lequel les particules sont paramagnétiques ou superparamagnétiques.

5. Dispositif médical pour l'utilisation selon l'une des revendications précédentes, dans lequel
i) le dispositif médical est imprégné, revêtu ou imbibé des particules magnétiques, et/ou
ii) le dispositif médical est biologiquement résorbé en 1 à 12 mois, et/ou
iii) le dispositif médical est physiologiquement compatible et est dégradé en constituants physiologiquement compatibles, et/ou
iv) le dispositif médical est flexible et n'est pas constitué d'un métal ou d'un alliage métallique, et le dispositif médical ne se présente pas sous la forme d'une solution aqueuse des particules.

6. Dispositif médical pour l'utilisation selon l'une des revendications 3 à 5, le dispositif médical biodégradable libérant les particules magnétiques et/ou les cédant au tissu tumoral environnant ou aux cellules tumorales environnantes.

7. Dispositif médical pour l'utilisation selon l'une des revendications précédentes, dans lequel les particules magnétiques sont contenues selon une concentration de 10 µg à 100 mg par cm² d'aire du dispositif médical, et/ou dans lequel les particules magnétiques sont contenues en une concentration de 100 µg à 2 g par g du dispositif médical.

8. Dispositif médical pour l'utilisation selon l'une des revendications précédentes, dans lequel, pour ce qui concerne le dispositif médical, il s'agit de cellulose à usage médical, de matériaux pour pansements, de garnitures de plaies, d'un matériel de suture chirurgicale, de compresses, d'éponges, de textiles à usage médical ou de compositions filmogènes.

9. Dispositif médical pour l'utilisation selon l'une des revendications précédentes, le dispositif médical contenant en outre au moins une substance thérapeutiquement efficace, choisie dans le groupe comprenant les principes actifs antiprolifératifs, anti-migration, anti-angiogènes, antithrombotiques, anti-inflammatoires, antiphlogistiques, cytostatiques, cytotoxiques, anticoagulants, antibactériens, antiviraux et/ou antimycotiques, en particulier dans lequel la ou les substances thérapeutiques sont choisies dans le groupe comprenant l'actinomycine D, l'aminoglutéthimide, l'amsacrine, l'anastrozol, les antagonistes des bases puriques et pyrimidiques, les anthracyclines, les inhibiteurs d'aromatases, l'asparaginase, les anti-oestrogènes, le bexarotène, la bléomycine, la busélérine, le busulfan, les dérivés de camptothécine, la capécitabine, le carboplatine, la carmustine, le chlorambucil, le cisplatine, la cladribine, le cyclophosphamide, la cytarabine, le cytosine-arabinoside, les cytostatiques alkylants, la dacarbazine, la daclinomycine, la daunorubicine, le docétaxel, la doxorubicine, l'épirubicine, l'estramustine, l'étoposide, l'exémestan, la fludarabine, le fluorouracile, les antagonistes de l'acide folique, le formestan, la gemcitabine, les glucocorticoïdes, la gosalérine, les hormones et antagonistes des hormones, l'hycamtine, l'hydroxy-urée, l'idarubicine, l'ifosfamide, l'imatinib, l'irinotécan, le létrozol, la leuproréline, la lomustine, le melphalan, la mercaptopurine, le méthotréxate, la miltéfosine, la mitomycine, les inhibiteurs de mitose, le mitoxantron, la nimustine, l'oxaliplatine, le paclitaxel, la pentostatine, la procarbazine, le tamoxifène, le témozolomide, le téniposide, la testolactone, le thiotépa, la tioguanine, les inhibiteurs de topoisomérase, le topotécan, le tréosulfan, la trétinoïne, la triptoréline, le trofosfamide, la vinblastine, la vincristine, la vindésine, la vinorelbine, les antibiotiques à effet cytostatique.

10. Dispositif médical pour l'utilisation selon la revendication 9, dans lequel au moins un principe actif est lié à la particule par adhérence, par liaison ionique, par liaison covalente ou par l'intermédiaire d'un lieur.

11. Dispositif médical pour l'utilisation selon l'une des revendications 9 ou 10, dans lequel le détachement du ou des principes actifs, à partir du support, est déclenché par un champ magnétique alternatif.

12. Dispositif médical pour l'utilisation selon l'une des revendications précédentes, le dispositif médical étant constitué du matériau suivant : poly(acide acrylique), polyacrylates, poly(méthacrylate de méthyle), poly(méthacrylate de butyle), poly(méthacrylate d'isobutyle), polyacrylamide, polyacrylonitrile, polyamide, polyétheramide, polyéthylène-amine, polyimide, polycarbonate, polycarbo-uréthanne, polyvinylcétone, poly(halogénure de vinyle), poly(halogénure de vinylidène), polyvinyléther, composés polyvinylaromatiques, poly(ester vinylique), polyvinylpyrrolidone, polyoxyméthylène, polyéthylène, polypropylène, polytétrafluoréthylène, polyuréthanne, élastomère de polyoléfine, polyisobutylène, caoutchoucs EPDM, fluorosilicone, carboxyméthylchitosan, poly(téréphtalate d'éthylène), polyvalérate, carboxyméthylcellulose, cellulose, rayonne, triacétate de rayonne, nitrate de cellulose, acétate de cellulose, hydroxyéthylcellulose, butyrate de cellulose, acétobutyrate de cellulose, copolymère d'acétate d'éthylvinyle, polysulfone, polyéthersulfone, résine époxy, résines ABS, caoutchoucs EPDM, prépolymère de silicone, silicone, polysiloxane, composé polyvinylhalogéné, éther de cellulose, triacétate de cellulose, chitosan, dérivés de chitosan, huiles polymérisables, polyvalérolactones, poly-ε-décalactone, polylactide, polyglycolide, copolymères des polylactides et des polyglycolides, poly-ε-caprolactone, poly(acide hydroxybutyrique), polyhydroxybutyrate, polyhydroxyvalérate, poly(hydroxybutyrate-co-valérate), poly(1,4-dioxanne-2,3-dione), poly(1,3-dioxann-2-one), polyparadioxannone, polyanhydride, poly(anhydride maléique), polyhydroxyméthacrylate, polycyanoacrylate, poly(diméthacrylate de caprolactone), poly(acide p-maléique), poly(acrylate de caprolactonebutyle), copolymères multiblocs d'oligocaprolactonediol et d'oligodioxanonediol, polymères multiblocs de polyétheresters de PEG et de poly(téréphtalate de butylène), polypivololactone, poly(acide glycolique-triméthylcarbonate), polycaprolactoneglycolide, poly(y-glutamate d'éthyle), poly(iminocarbonate de DTH), poly(carbonate de DTE-co-DT), poly(bisphénol A-iminocarbonate), polyorthoester, poly(acide glycolique-triméthylcarbonate), poly(triméthylcarbonate), polyiminocarbonate, poly(alcools vinyliques), polyestéramides, polyesters glycolés, polyphosphoesters, polyphosphazènes, poly[p-(carboxyphénoxy)propane], poly(acide hydroxypentanoïque), poly(oxyde d'éthylène-oxyde de propylène), polyuréthannes souples, polyuréthannes ayant des résidus acides aminés dans le squelette, polyétherester, poly(oxyde d'éthylène), poly(oxalates d'alcène), polyorthoesters, carragénanes, amidon, collagène, polymères à base de protéines, poly(acides aminés), poly(acides aminés synthétiques), zéine, zéine modifiée, polyhydroxyalcanecétène, acide pectique, acide actinique, fibrine, fibrine modifiée, caséine, caséine modifiée, carboxyméthylsulfate, albumine, acide hyaluronique, hépéranesulfate, héparine, chondroïtinesulfate, dextran, cyclodextrines, copolymères de PEG et de polypropylèneglycol, gomme arabique, gomme de guar ou d'autres résines de type gomme, gélatine, collagène, collagène-N-hydroxysuccinimide, lipides, lipoïdes, huiles polymérisables et leurs modifications, copolymères et mélanges des substances mentionnées ci-dessus.

13. Dispositif médical pour l'utilisation selon l'une des revendications précédentes, pour lequel le cancer, la tumeur ou la maladie proliférative est choisi dans le groupe comprenant les adénocarcinomes, le mélanome de la choroïde, la leucémie aiguë, le neurinome de l'acoustique, le carcinome de l'ampoule, le carcinome anal, les astrocytomes, le basaliome, le cancer du pancréas, la tumeur du tissu conjonctif, le cancer de la vessie, le carcinome bronchique, le cancer des bronches non à petites cellules, le cancer du sein, le lymphome de Burkitt, le cancer du corps utérin, le syndrome CUP, le cancer du gros intestin, le cancer de l'intestin grêle, les tumeurs de l'intestin grêle, le cancer des ovaires, le carcinome de l'endomètre, l'épendymome, les types de cancers de l'épithélium, les tumeurs d'Ewing, les tumeurs gastro-intestinales, le cancer de la vésicule biliaire, les carcinomes biliaires, le cancer de l'utérus, le cancer du col de l'utérus, les glioblastomes, les tumeurs gynécologiques, les tumeurs du cou, du nez et des oreilles, les néoplasies hématologiques, le cancer de l'urètre, le cancer de la peau, les tumeurs cérébrales (gliomes), les métastases cérébrales, le cancer des testicules, les tumeurs de l'hypophyse, les carcinoïdes, le sarcome de Kaposi, le cancer du larynx, la tumeur des cellules germinales, le cancer des os, le carcinome colorectal, les tumeurs de la tête et du cou (tumeurs de la zone ORL), le cancer du côlon, les craniopharyngéomes, le cancer dans la zone de la bouche et sur la lèvre, le cancer du foie, les métastases hépatiques, la tumeur des paupières, le cancer du poumon, le cancer des ganglions lymphatiques (hodgkinien/non hodgkinien), les lymphomes, le cancer de l'estomac, le mélanome malin, le néoplasme malin, les malignomes du tractus gastro-intestinal, le cancer mammaire, le cancer du rectum, les médulloblastomes, le mélanome, les méningéomes, la maladie de Hodgkin, la mycose fongoïde, le cancer du nez, le neurinome, le neuroblastome, le cancer du rein, le cancer des cellules rénales, les lymphomes non-hodgkiniens, l'oligodendrogliome, le cancer de l'oesophage, les carcinomes ostéolytiques et les carcinomes ostéoplasiques, l'ostéosarcome, le cancer ovarien, le cancer du pancréas, le cancer du pénis, les carcinomes malpighiens de la tête et du cou, le cancer de la prostate, le cancer du pharynx, le cancer du rectum, le rétinoblastome, le cancer du vagin, le cancer de la thyroïde, la maladie de Schneeberger, le cancer de l'oesophage, le spinaliome, le lymphome à cellules T (mycose fongoïde), le thymome, le cancer des trompes de Fallope, les tumeurs de l'oeil, le cancer de l'urètre, les tumeurs urologiques, le carcinome urothélial, le cancer de la vulve, l'apparition de verrues, les tumeurs des parties molles, le sarcome des parties molles, la tumeur de Wilms, le cancer du col de l'utérus et le cancer de la langue.

14. Dispositif médical pour l'utilisation selon l'une des revendications précédentes, pour lequel le post-traitement du domaine opératoire sert à empêcher la formation de récidives.

15. Utilisation d'un tissu, d'une éponge ou d'un film physiologique, solide ou en gel, pouvant être chauffé sous l'effet d'un champ magnétique alternatif, pour la fabrication d'un dispositif médical destiné au post-traitement du domaine opéré lors d'opérations du cancer, pour laquelle des particules magnétiques sont contenues dans le dispositif médical, qui sont excitées par un champ magnétique alternatif, produisent de la chaleur et de ce fait chauffent le dispositif médical.
